# EUROPEAN PATENT APPLICATION

(11) **EP 3 275 446 A1**
(43) Date of publication of application: **31.01.2018**
(21) Application number: 16180950.4
(22) Date of filing: 25.07.2016
(51) Int. Cl.: A61K 31/702, A61K 36/53

(54) **PREBIOTIC COMPOSITIONS COMPRISING OLIGOSACCHARIDES FROM HYPTIS SUAVEOLENS**

(71) Applicant: Sinutron KG, 1180 Vienna (AT)
(72) Inventor: PRAZNIK, Werner, 1180 Vienna (AT); OKONOGI, Siriporn, 1180 Vienna (AT); UNGER, Frank Michael, 1180 Vienna (AT); VIERNSTEIN, Helmut, 1180 Vienna (AT); HOLZER, Wolfgang, 1180 Vienna (AT); MÜLLER, Monika, 1180 Vienna (AT); CAVARKAPA, Andrea, 1180 Vienna (AT)
(74) Representative: Sonn & Partner Patentanwälte

(57) **Abstract**

Disclosed is a prebiotic composition comprising one or more oligosaccharides, selected from the group consisting of
- a βGlc-(1→4) [βGal-(1→2)-α-Gal-(1→6)] Man tetrasaccharide,
- a nonasaccharide consisting of a Glc(1→ ) βMan(1→4)βGlc(1→4)[βGal (1→2)αGal(1→6)]Man hexasaccharide with three βGal monosaccharide residues attached as branches to the hexasaccharide, and
- higher oligosaccharides (degree of polymerization 9 - 13), typically consisting of a Glc(1→) Glc(1→) Glc(1→) βMan(1→4)βGlc(1→4) [βGal(1→2)αGal(1→6)]Man with three or more β-Gal monosaccharide residues attached as branches to the octasaccharide,
wherein the tetrasaccharide and/or the nonasaccharide and/or the higher oligosaccharides is/are contained in the composition in an amount of at least 5 % w/w, preferably at least 10 % w/w, especially at least 25 % w/w, dry weight.

## Description

The present invention relates to prebiotic compositions, methods for producing such compositions and uses thereof.

"A prebiotic is a selectively fermented ingredient that allows specific changes, both in the composition and/or activity in the gastrointestinal microflora that confers benefits upon host well-being and health" (Roberfroid M. B., "Prebiotics: the concept revisited" Journal of Nutrition, 137, (3 suppl. 2) 830S - 837 S PMID 17311983).

Prebiotics are specifically targeted to act on the flora in the large intestine. It is "assumed that a prebiotic should increase the numbers and/or activities of bifidobacteria and lactobacilli".

Among the benefits upon host well-being and health, the following effects of prebiotics have been claimed
- Relief of constipation
- Reduction of the intestinal pH-values
- Restoration of the intestinal bacterial balance
- Beneficial effects on blood cholesterol levels
- Reduction of the risk of colorectal cancer
- Beneficial effects on the immune system
- Improved quality of the intestinal flora in infants.

To be considered a true prebiotic, a substance has to fulfil several criteria (Gibson GR, Roberfroid MB 1995. Dietary modulation of the human colon microbiota: introducing the concept of prebiotics, Journal of Nutrition, 125, 1401 - 1412):
- Resists gastric acidity
- Is not hydrolyzed by GI tract enzymes
- Is not absorbed in the upper GI tract
- Is fermented by intestinal microorganisms
- Induces selective stimulation of growth and/or activity of intestinal bacteria, potentially associated with health or well-being.

Presently there are only two food ingredients that fulfil these criteria: inulin and transgalactooligosaccharides.

Inulin belongs to the class of polysaccharides known as fructans; it is made up mostly of β-fructofuranosyl units, has a degree of polymerization of ca. 35, a molecular mass in the range of 5000, and it partially or completely replaces starch as a reserve food in *Compositae.* Inulin may be isolated from Jerusalem artichoke tubers and from other sources (e.g. Cichorium intybus, chicory).

Inulin can be degraded enzymatically or chemically into a mixture of oligosaccharides termed fructooligosaccharides (FOS) with a chain length ranging from 1 to 7 glycosyl units. Fructooligosaccharides can be produced not only by degradation of inulin but also by enzyme-catalyzed synthesis from sucrose.

Galactooligosaccharides are produced from lactose by the action of β-galactosidases which have transgalactosylation activity; tri- to hexasaccharides with 2 - 5 galactose units are the main products of the reactions (T. Sako, K. Matsumoto, R. Tanaka, Recent Progress on research and applications of non-digestible galacto-oligosaccharides, International Dairy Journal 9 (1999) 69-80). Galactooligosaccharides are used as food additives in beverages, yoghurts and yoghurt drinks. Other applications are desserts such as ice creams and jellies, biscuits, breads, pastries, jams and marmalades. A frequent use of galactooligosaccharides is in infant formulas. Non-food applications have been suggested in drug delivery, cosmetics and mouth washes.

It is an object of the present invention to provide improved prebiotic compositions with superior properties, as well as methods for the production of such compositions. The compositions should be suitable for prebiotic uses and comply with the criteria to be fulfilled by "true prebiotics".

Therefore, the present invention provides a prebiotic composition comprising one or more oligosaccharides, selected from the group consisting of
- a βGlc-(1→4) [βGal-(1→2)-α-Gal-(1→6) ] Man tetrasaccharide,
- a nonasaccharide consisting of a Glc(1→ ) βMan(1→4)βGlc(1→4) [βGal (1→2)αGal(1→6)]Man hexasaccharide with three βGal monosaccharide residues attached as branches to the hexasaccharide, and
- higher oligosaccharides with a degree of polymerization 9 - 13, typically consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide,
wherein the tetrasaccharide and/or the nonasaccharide and/or the higher oligosaccharides is/are contained in the composition in an amount of at least 5 % w/w, preferably at least 10 % w/w, especially at least 25 % w/w, dry weight (all % w/w values given herein are - except indicated otherwise - 5 w/w of dry weight).

In the course of the present invention it was revealed that galactooligosaccharides promoting the growth of beneficial lactobacilli and bifidobacteria can be produced not only by transglycosylation from lactose, but also by enzyme-catalyzed degradation of certain galactoglucomannans that are richly decorated with β-galactopyranosyl branches in the form of monosaccharide stubs. As distinct from most of the known galactoglucomannans, the parent polysaccharide from which the oligosaccharides of the invention are derived has a high content of β-galactopyranosyl units, so that galactose is by far the preponderant monosaccharide constituent of that polysaccharide.

If the composition according to the present invention is prepared by using *Hyptis suaveolens* seeds as starting material, the composition is further characterized by a specific ratio of Gal : Glc : Man saccharide moieties (as a kind of "fingerprint"), because such ratio of the parent polysaccharide is also present in the composition made from this starting material. Indeed, the ratio Gal : Glc : Man in the parent polysaccharide is ca. 9 : 5 : 3. By contrast, in galactoglucomannans from gymnosperms, it is commonly ca. 1 : 1 : 3, in the endosperm of the Judas tree, it is 2 : 1 : 11, in the endosperm of asparagus seeds, it is 1 : 4 : 5, and in poplar wood, 1 : 4 : 10. Not only do these galactoglucomannans contain less galactose, but the Gal units they contain are mostly in the α-anomeric configuration which is less advantageous for use in prebiotic preparations.

According to a preferred embodiment, the oligosaccharides of the invention are therefore characterized by ratios of Gal : Glc : Man monosaccharide compositions ranging from 2 : 1 : 1 (via further experimentally detected ratios, such as 2.5 : 1 : 1) to 3.2 : 2.6 : 1. Based on the use of *Hyptis suaveolens* seeds as starting material, this starting material is also reflected in the respective composition ratios of the oligosaccharides in the prebiotic composition according to the present invention ranging from 2 : 1 : 1 (via e.g. 2.5 : 1 : 1) to 3.2 : 2.6 : 1 with galactose always the preponderant constituent sugar. In the lower oligosaccharides (degree of polymerization 4 - 9) the ratio of Glc : Man is 1 : 1 whereas in the higher oligosaccharides (degree of polymerization >9) glucose preponderates over mannose up to 2.5 fold. A preferred embodiment of the present invention is therefore a composition which is obtained from mucilage of *Hyptis suaveolens* seeds and therefore has a specific ratio of Gal : Glc : Man saccharide moieties of about 9 : 5 : 3.

The function of galactooligosaccharides as carbon sources for lactobacilli and bifidobacteria involves the release, by the action of exo-galactosidase enzymes, of a non-reducing-terminal galactose unit, so that the free sugar galactose can be metabolized by the bacteria. In conventional transgalactooligosaccharides, the β-galactopyranosyl residues are arranged mostly in linear chains, so that the release of the galactose sugar molecules occurs sequentially. By contrast, in the galactooligosaccharides of the present invention, the β-galactopyranosyl residues are attached to the glucomannan cores mostly in the form of lateral monosaccharide stubs, so that lactobacilli or bifidobacteria equipped with β-galactosidase activity can effect the simultaneous release of most of the galactose.

As distinct from α-galactooligosaccharides and similar to other β-galactooligosaccharides, the galactooligosaccharides of the present invention are expected to cause minimal gas formation and bloating [Rycroft, CE, Jones MR, Gibson GR, Rastall RA, A comparative in vitro evaluation of the fermentation properties of prebiotic oligosaccharides, J. Appl. Microbiol. 91, 878 - 887 (2001)].

The prebiotic preparations according to the present invention therefore comprise advantageous properties as prebiotics. The compositions according to the present invention are freely soluble in water and will not give rise to gel formation with other polysaccharides such as pectins. The compositions according to the present invention are better degradable (especially better than polysaccharide containing preparations) due to their oligosaccharide nature. The oligosaccharides of the invention are better accessible by synthetic chemistry such as combined organic-chemical and enzyme-catalyzed synthesis.

The present compositions are also superior to conventional transgalactooligosaccharides as well as to galactomannan oligosaccharides and soybean-oligosaccharides, because in conventional galactooligosaccharides the single galactose units (although also present in β-linkage) are bound sequentially as Gal-Gal-Gal etc. chains. In the composition according to the present invention, however, the galactose units are bound individually to a glucose-mannose backbone and have therefore a better bioavailability (because cleavage by bacterial enzymes is facilitated). In galactomannan oligosaccharides and soybean-oligosaccharides galactose is present mainly in the α-linkage. These oligosaccharides, as well as fructooligosaccharides and inulin, lead to increased flatulence and are therefore not within the meaning of the FODMAP concept. This concept (Fermentable Oligosaccharides, Disaccharides, Monosaccharides And Polyols) was developed in Australia in response to the increased incidence of irritable bowel syndrome. This syndrome is characterized by an increased sensitivity to distension stimuli in the intestine; therefore prebiotics, which cause flatulence, can result in cramps and pain. β-Linked galactooligosaccharides as contained in the present composition do explicitly not fall under FODMAP. According to a preferred embodiment, the compositions according to the present invention are therefore essentially free (i.e. trace amounts at the most) of conventional galactomannan oligosaccharides and soybeans-oligosaccharides wherein the galactose units (although also present in β-linkage) are bound sequentially as e.g. Gal-Gal-Gal (or Gal-Gal-Gal-Gal, etc.) chains.

The prebiotic oligosaccharides according to the present invention are advantageous as carbon sources to support the growth of probiotic bacteria in culture. Probiotic bacteria, mostly of the genus lactobacilli and bifidobacteria, are those that exert beneficial effects in a mammalian host when administered in sufficient amounts.

The prebiotic composition according to the present invention can preferably further comprise oligosaccharides having growth-promoting activity toward one or more of certain lactobacilli or bifidobacteria, and containing as monosaccharide constituents glucose, galactose, mannose, xylose, arabinose, fucose, galacturonic acid, glucuronic acid, and/or 4-methoxyglucuronic acid.

Preferred compositions according to the present invention comprise oligosaccharides containing between three and twelve glycosyl residues (glycosyl units), preferably composed of a glucomannan core (backbone) of the type βGlc- (1→4) - βMan- (1→4) - βGlc-(1→4)-Man, alternatively containing two or more contiguous glucose units as in (Glc 1→ ) Glc (1→4) βGlc (1→4) βMan (1→4)βGlc (1→4)Man.

The compositions according to the present invention are preferably free of polysaccharides, i.e. do not contain saccharide chains longer than thirteen monosaccharides covalently linked in amounts higher than trace amounts (for example, the compositions according to the present invention contain less than 5% w/w polysaccharides, preferably less than 1 % w/w, especially less than 0.5 % w/w), whereas the compositions according to the present invention contain up to 100 % (of all saccharide molecules) oligosaccharides consisting of three to thirteen monosaccharides (glycosyl units). In other embodiments, also mono- and disaccharides may be contained in the compositions according to the present invention, even above trace amounts.

Accordingly, the compositions according to the present invention consist preferably of 50 % w/w or more, more preferred of 70 % w/w or more, even more preferred of 90 % or more, especially of 95 % w/w or more, oligosaccharides consisting of three to thirteen monosaccharides (glycosyl units).

If the compositions of the present invention also contain mono- or disaccharides, they are preferably contained in an amount of below 30 % w/w, more preferred in an amount of below 10 % w/w, especially below 5 % w/w.

It is specifically preferred that the compositions according to the present invention consist preferably of 50 % w/w or more, more preferred of 70 % w/w or more, even more preferred of 90 % or more, especially of 95 % w/w or more, of oligosaccharides between three and thirteen monosaccharides with a βGlc-(1→4)Man group, preferably as branched oligosaccharides.

Since the preferred polysaccharides from which the present compositions are produced are irregular, i.e. there are core regions with more Glc than Man (see e.g. Example 7), in some oligosaccharides, there may be contiguous Glc sequences such as βGlc-(1→4)-βGlc-(1→4)-Man, etc.. Preferably, not only the tetrasaccharide and the nonasaccharide according to the present invention are branched (i.e. always having a "non(1→4)" bond to Glc, Man, (or Glc-Glc), i.e. an (1→2)-, (1→3)-, or (1→6)-bond), but also other oligosaccharides in the composition according to the present invention. Most of the branches are βGal-(1→ (monosaccharide stubs at 2, 3, 6; as indicated by 13C-NMR; see examples; not all βGal residues are necessarily linked to position 6 of Man. Some branches may be βGal-(1→2) -αGal-(1→ (6 Man), as in the tetrasaccharide and the nonasaccharide, but the αGal residues are in a minority (one each in the tetrasaccharide or nonasaccharide).

The prebiotic oligosaccharides according to the present invention (composed of glucose, galactose and mannose) can e.g. be prepared from the neutral polysaccharide of *Hyptis suaveolens* seeds. Aqueous workup of *H. suaveolens* seeds provides a mucilage composed mostly of polysaccharides and other components such as proteins. According to a preferred embodiment, the present prebiotic composition therefore comprises a hydrolysate of one or more heteropolysaccharides from plants, especially from plant seeds. Preferably, the present prebiotic compositions comprise a hydrolysate of one or more neutral heteropolysaccharides from plant seeds of *Hyptis suaveolens.*

The prebiotic composition according to the present invention is preferably produced as an enzyme-catalyzed hydrolysate, especially an endo-β-mannanase catalyzed hydrolysate, of one or more heteropolysaccharides from plants, especially from plant seeds.

The prebiotic composition according to the present invention preferably contains to a high amount (e.g. more than 50 % of all oligosaccharides) oligosaccharides that mainly consist of glucose, galactose and mannose.

According to a preferred embodiment, the compositions according to the present invention may be synbiotic, i. e. may contain probiotic bacteria in addition to oligosaccharides, preferably lactic acid bacteria, preferably probiotic lactobacilli and/or bifidobacteria; and/or neutral polysaccharides from *Hyptis suaveolens* seeds; and/or further prebiotic compounds, especially inulin and/or transgalactooligosaccharides. Furthermore, compositions may be combinations of prebiotics containing in addition neutral polysaccharide.

According to a preferred embodiment, the prebiotic composition according to the present invention may contain natural antioxidants such as flavonoid glycosides. According to another aspect, the present invention relates to a method for producing a prebiotic composition comprising the following steps:
- providing a mucilage of *Hyptis suaveolens* seeds;
- treatment of the mucilage with an alkaline agent, preferably a KOH or NaOH containing agent, thereby obtaining a supernatant and an insoluble part;
- separating the supernatant from the insoluble part, preferably by centrifugation, wherein the supernatant contains acidic and neutral polysaccharides;
- neutralizing the supernatant, preferably to a pH of 6 to 8;
- separating the neutral polysaccharide from the acidic polysaccharides, preferably by a chromatographic method, especially by use of an anion exchange resin, thereby obtaining the neutral polysaccharides;
- incubation of the neutral polysaccharides with *endo*-β-mannanase, thereby obtaining a composition comprising oligosaccharides.

The release of the mucilage from seeds of *Hyptis suaveolens* is performed by aqueous extraction using techniques well known in the art.

Preferably, the method provides a composition comprising oligosaccharides containing one or more oligosaccharides, selected from the group consisting of
- a βGlc-(1→4) [βGal-(1→2)-α-Gal-(1→6) ] Man tetrasaccharide,
- a nonasaccharide consisting of a Glc(1→ ) βMan(1→4)βGlc(1→4) [βGal (1→2)αGal(1→6)]Man hexasaccharide with three βGal monosaccharide residues attached as branches to the hexasaccharide, and
- higher oligosaccharides with a degree of polymerization 9 - 13, typically consisting of a Glc(1→ ) ) Glc(1→ ) ) Glc(1→ ) βMan(1→4)βGlc(1→4) [βGal (1→2)αGal(1→6)]Man with three or more β-Gal monosaccharide residues attached as branches to the octasaccharide.

The treatment of the mucilage is preferably performed with an NaOH suspension as alkaline agent, preferably containing 1 to 6 M NaOH, especially 2 to 5 M NaOH.

Preferably, the treatment of the mucilage with alkaline agent is performed at temperatures of between 40 and 90°C, preferably of 50 to 70°C, especially of 55 to 65°C.

Preferably, the treatment of the mucilage with alkaline agent is performed for 5 to 100 h, preferably for 10 to 80 h, especially of 24 to 72h.

According to a preferred embodiment, the separating of the supernatant from the insoluble part is performed by centrifugation and wherein the insoluble part is washed with an alkaline agent so as to obtain a further supernatant containing acidic and neutral polysaccharides and wherein this further supernatant may be combined with the supernatant or subjected independently to the further steps.

Preferably, the neutralized supernatant is subjected to a dialysis, especially a dialysis against distilled water.

According to a preferred embodiment, separating the neutral polysaccharide from the acidic polysaccharides is performed by anion exchange chromatography on a resin comprising DEAE groups, especially on DEAE cellulose, and in the presence of a buffer, preferably a TRIS/citrate buffer or a phosphate buffer.

Preferably, the incubation of the neutral polysaccharides with endo-β-mannanase is performed at a temperature of 20 to 60°C, preferably at a temperature of 30 to 55°C, especially at a temperature of 40 to 50°C.

Preferably, the mannanase usable in the present invention is a purified enzyme essentially without other polysaccharide degrading capacity than endo-β-mannanase activity. A specifically preferred β-mannanase used in the method according to the present invention is the high-purity endo-1,4-β-mannanase from *Aspergillus niger* (EC 3.2.1.78), especially the enzyme supplied by Megazyme, Bray, Co. Wicklow, Ireland.

Preferably, the incubation of the neutral polysaccharides with endo-β-mannanase is performed for 12 h to 20 d, preferably for 1 d to 10 d, especially for 2 d to 5 d.

According to a preferred embodiment, the prebiotic composition according to the present invention is a preparation obtainable by the method according to the present invention. This composition is characterized by a specific pattern of oligosaccharides which enables superior prebiotic properties, as e.g. evidenced by the representative assays disclosed in the present application, especially Example 5.

According to another aspect, the prebiotic composition according to the present invention is used as a food supplement or functional food.

According to a further aspect, the present invention relates to the present prebiotic composition for use in the prevention or treatment of intestinal disorders, preferably of constipation, increased blood cholesterol levels, distorted balance of intestinal bacterial flora, increased intestinal pH values, and inflammatory bowel diseases.

Preferred embodiments of the present composition may therefore further comprise pharmaceutically active substances for ameliorating irritable bowel syndrome or inflammatory bowel syndrome, for example stool-softeners, laxatives, serotonin agonists, tricyclic antidepressants, immunosuppressive, anti-inflammatory steroids and antispasmodics. Accordingly, the prebiotic compositions according to the present invention may additionally comprise pharmaceutically active substances for ameliorating irritable bowel syndrome that are selected from the group of ondansetron, clozapine, olanzapine, hyoscyamine, dicyclomine, phenobarbitals, especially donnatal and atropine, musculotropics, especially mebeverine, tegaserod, magnesium aluminum silicates, alverine citrate drugs, rifaximin, opiates, especially morphine and oxycodone, opioids, opioid analogs, especially loperamide, codeine, diphenoxylate; mesalazine, prednisone, TNF inhibitors, azathioprine, methotrexate, and 6-mercaptopurine.

According to a further aspect, the present invention also relates to new oligosaccharides, namely,
- a βGlc-(1→4) [βGal-(1→2)-α-Gal-(1→6) ] Man tetrasaccharide,
- a nonasaccharide consisting of a Glc(1→ ) βMan(1→4)βGlc(1→4) [βGal (1→2)αGal(1→6)]Man hexasaccharide with three βGal monosaccharide residues attached as branches to the hexasaccharide, and
- higher oligosaccharides with a degree of polymerization 9 - 13 typically consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide.

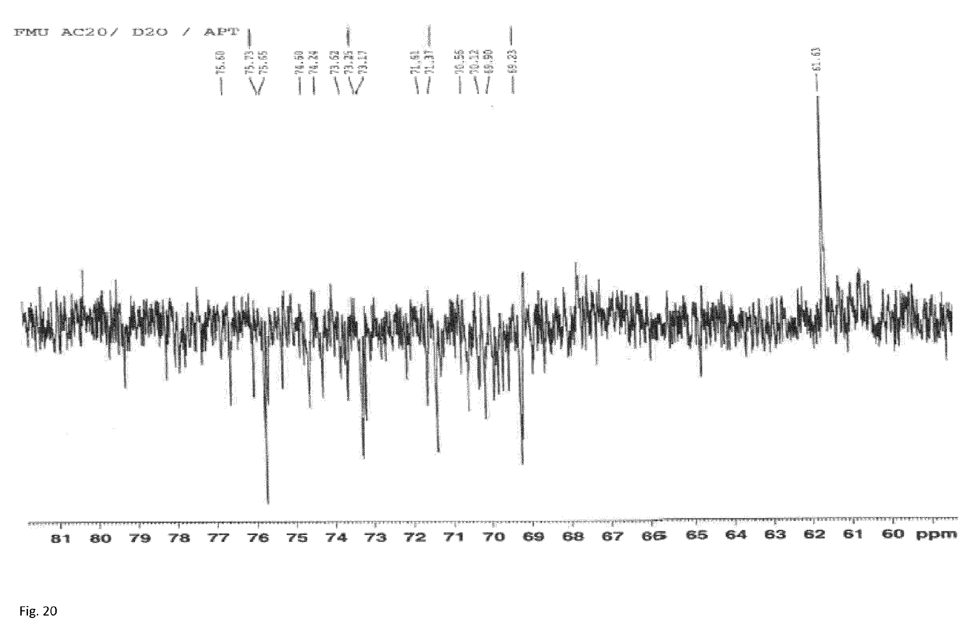

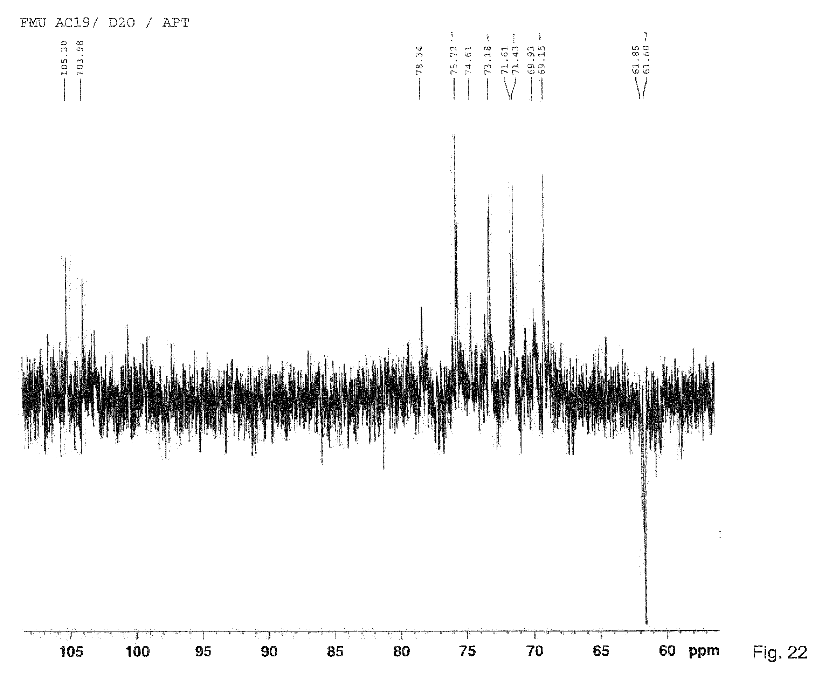

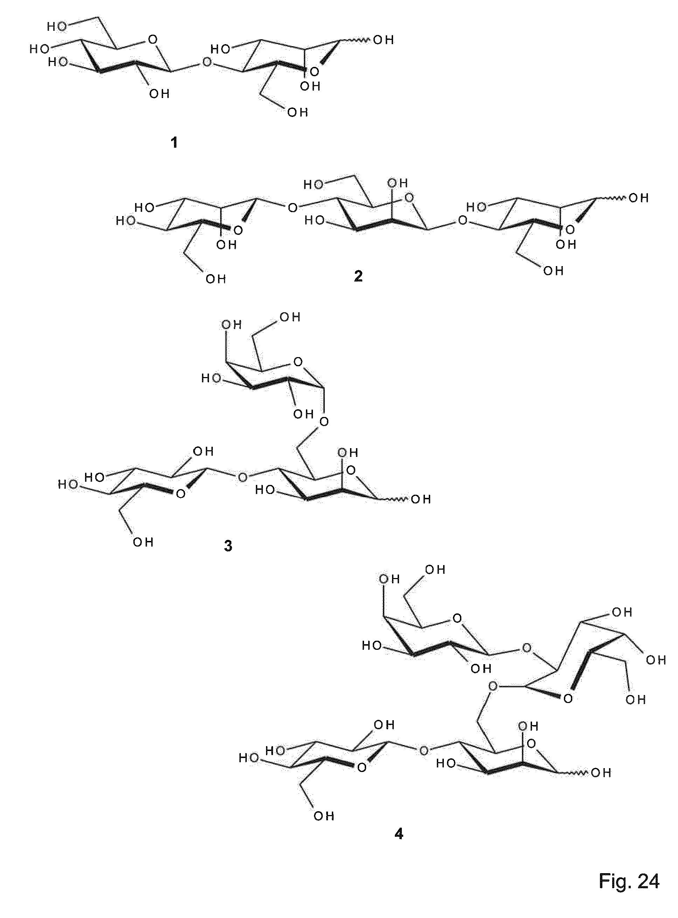

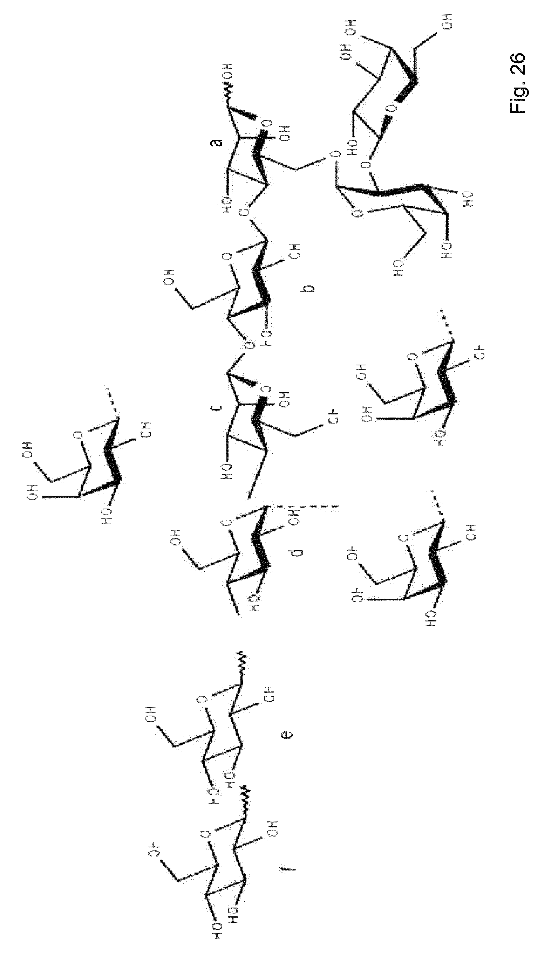

These oligosaccharides have specifically preferred properties as prebiotic substances, as evidenced with the present invention, especially with Example 5 herein. These oligosaccharides, especially the nonasaccharide are obtainable by a method as disclosed herein (i.e. by production from *Hyptis suaveolens* seeds), however, they can also be produced by chemical synthesis or by processing from other natural or synthetic polysaccharides. Whereas the structure of the tetrasaccharide is clear, the structure of the nonasaccharide obtainable by the present invention has not yet been finally determined in an exact manner. It has been confirmed experimentally in the course of the present invention by using Dionex analysis that the nonasaccharide has the composition Gal: Glc: Man 5: 2: 2. This implies a Glc-Man-Glc-Man backbone, which is "decorated" with an α-galactose and four β-galactoses (see Fig. 24). Similarly, the preparations of the invention may contain higher oligosaccharides with dp 10 - 13 with similar bioactivity but with a typical Gal : Glc : Man ratio of 3.2 : 2.6 : 1. By ¹³C-NMR spectroscopy (Fig. 22), these oligosaccharides are decorated with at least three βGal monosaccharide branches similar to the nonasaccharide 5 (Fig. 20), but contain substantially more glucose residues in the core (Fig. 26).

Accordingly, the present invention is drawn to the following preferred embodiments:
1. Prebiotic composition comprising one or more oligosaccharides, selected from the group consisting of
   - a βGlc-(1→4) [βGal-(1→2)-α-Gal-(1→6) ] Man tetrasaccharide,
   - a nonasaccharide consisting of a Glc(1→ ) βMan(1→4)βGlc(1→4)[βGal (1→2)αGal(1→6)]Man hexasaccharide with three βGal monosaccharide residues attached as branches to the hexasaccharide, and
   - higher oligosaccharides with a degree of polymerization 9 - 13 consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide,
   wherein the tetrasaccharide and/or the nonasaccharide and/or the higher oligosaccharides is/are contained in the composition in an amount of at least 5 % w/w, preferably at least 10 % w/w, especially at least 25 % w/w, dry weight.
2. Prebiotic composition according to embodiment 1, further comprising a βGlc-(1→4)-[αGal-(1→6) ] Man trisaccharide, preferably in an amount of at least 5 % w/w, more preferred at least 10 % w/w, especially at least 25 % w/w, dry weight.
3. Prebiotic composition according to embodiment 1 or 2, further comprising a βGlc-(1→4)Man disaccharide, preferably in an amount of at least 5 % w/w, more preferred at least 10 % w/w, especially at least 25 % w/w, dry weight.
4. Prebiotic composition according to any one of embodiments 1 to 3, further comprising oligosaccharides having growth-promoting activity toward one or more of certain lactobacilli or bifidobacteria, and containing as monosaccharide constituents glucose, galactose, mannose, xylose, arabinose, fucose, galacturonic acid, glucuronic acid, and/or 4-methoxyglucuronic acid.
5. Prebiotic composition according to any one of embodiments 1 to 4, comprising a hydrolysate of one or more heteropolysaccharides from plants, especially from plant seeds.
6. Prebiotic composition according to any one of embodiments 1 to 5, comprising a hydrolysate of one or more neutral heteropolysaccharides from plant seeds of *Hyptis suaveolens.*
7. Prebiotic composition according to any one of embodiments 1 to 6, comprising an enzyme-catalyzed hydrolysate, especially an endo-β-mannanase catalyzed hydrolysate, of one or more heteropolysaccharides from plants, especially from plant seeds.
8. Prebiotic composition according to any one of embodiments 1 to 7, wherein the oligosaccharides essentially consisting of oligosaccharides that mainly consist of glucose, galactose and mannose.
9. Prebiotic composition according to any one of embodiments 1 to 8, further comprising probiotic bacteria, preferably probiotic lactobacilli and/or bifidobacteria; and/or neutral polysaccharides from *Hyptis suaveolens* seeds; and/or further prebiotic compounds, especially inulin and/or transgalactooligosaccharides.
10. Prebiotic composition according to any one of embodiments 1 to 9, having a ratio Gal : Glc : Man from 2 : 1 : 1 to 3.2 : 2.6 : 1, especially of about 9 : 5 : 3 or 2.5 : 1 : 1.
11. Prebiotic composition according to any one of embodiments 1 to 10, wherein polysaccharides are contained in an amount of less than 5 % w/w, preferably less than 1 % w/w, especially less than 0.5 % w/w).
12. Prebiotic composition according to any one of embodiments 1 to 11, wherein oligosaccharides between three and thirteen monosaccharides are contained of 50 % w/w or more, preferably of 70 % w/w or more, even more preferred of 90 % or more, especially of 95 % w/w or more.
13. Prebiotic composition according to any one of embodiments 1 to 12, wherein oligosaccharides consisting of three to thirteen monosaccharides with βGlc-(1→4)Man group are contained of 50 % w/w or more, preferably of 70 % w/w or more, even more preferred of 90 % or more, especially of 95 % w/w or more.
14. Prebiotic composition according to any one of embodiments 1 to 13, wherein branched oligosaccharides between three and thirteen monosaccharides with a βGlc-(1→4)Man group are contained of 50 % w/w or more, preferably of 70 % w/w or more, even more preferred of 90 % or more, especially of 95 % w/w or more.
15. Prebiotic composition according to any one of embodiments 1 to 13, wherein mono- or disaccharides are contained in an amount of below 30 % w/w, preferably in an amount of below 10 % w/w, especially below 5 % w/w.
16. Prebiotic composition according to any one of embodiments 1 to 15, wherein the composition is obtained from mucilage of *Hyptis suaveolens* seeds.
17. Prebiotic composition according to any one of embodiments 1 to 16, wherein the composition is essentially free of conventional galactomannan oligosaccharides and soybean-oligosaccharides, comprising sequential as Gal-Gal-Gal galactose units.
18. Prebiotic composition according to any one of embodiments 1 to 17, further comprising pharmaceutically active substances for ameliorating irritable bowel syndrome or inflammatory bowel syndrome.
19. Prebiotic composition according to embodiment 18, wherein the pharmaceutically active substances for ameliorating irritable bowel syndrome or inflammatory bowel syndrome are selected from the group of stool-softeners, laxatives, serotonin agonists, tricyclic antidepressants, immunosuppressives, anti-inflammatory steroids and antispasmodics.
20. Prebiotic composition according to embodiment 18 or 19, wherein the pharmaceutically active substances for ameliorating irritable bowel syndrome are selected from the group of ondansetron, clozapine, olanzapine, hyoscyamine, dicyclomine, phenobarbitals, especially donnatal and atropine, musculotropics, especially mebeverine, tegaserod, magnesium aluminum silicates, alverine citrate drugs, rifaximin, opiates, especially morphine and oxycodone, opioids, opioid analogs, especially loperamide, codeine, diphenoxylate; mesalazine, prednisone, TNF inhibitors, azathioprine, methotrexate, and 6-mercaptopurine.
21. Method for producing a prebiotic composition comprising the following steps:
   - providing a mucilage of *Hyptis suaveolens* seeds;
   - treatment of the mucilage with an alkaline agent, preferably a KOH or NaOH containing agent, thereby obtaining a supernatant and an insoluble part;
   - separating the supernatant from the insoluble part, preferably by centrifugation, wherein the supernatant contains acidic and neutral polysaccharides;
   - neutralizing the supernatant, preferably to a pH of 6 to 8;
   - separating the neutral polysaccharide from the acidic polysaccharides, preferably by a chromatographic method, especially by use of an anion exchange resin, thereby obtaining the neutral polysaccharides;
   - incubation of the neutral polysaccharides with *endo*-β-mannanase, thereby obtaining a composition comprising oligosaccharides.
22. Method according to embodiment 21, wherein the composition comprising oligosaccharides obtained contains one or more oligosaccharides, selected from the group consisting of
   - a βGlc-(1→4) [βGal-(1→2)-α-Gal-(1→6) ] Man tetrasaccharide,
   - a βGlc-(1→4)-[αGal-(1→6)]Man trisaccharide
   - a nonasaccharide consisting of a Glc(1→ ) βMan(1→4)βGlc(1→4)[βGal (1→2)αGal(1→6)]Man hexasaccharide with three βGal monosaccharide residues attached as branches to the hexasaccharide, and
   - higher oligosaccharides with a degree of polymerization 9 - 13 consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide.
23. Method according to embodiment 21 or 22, wherein the treatment of the mucilage with alkaline agent is performed in an NaOH suspension, preferably containing 1 to 6 M NaOH, especially 2 to 5 M NaOH.
24. Method according to any one of embodiments 21 to 23, wherein the treatment of the mucilage with alkaline agent is performed at temperatures of between 40 and 90°C, preferably of 50 to 70°C, especially of 55 to 65°C.
25. Method according to any one of embodiments 21 to 24, wherein the treatment of the mucilage with alkaline agent is performed for 5 to 100 h, preferably for 10 to 80 h, especially of 24 to 72h.
26. Method according to any one of embodiments 21 to 25, wherein the separating of the supernatant from the insoluble part is performed by centrifugation and wherein the insoluble part is washed with an alkaline agent so as to obtain a further supernatant containing acidic and neutral polysaccharides and wherein this further supernatant may be combined with the supernatant or subjected independently to the further steps.
27. Method according to any one of embodiments 21 to 26, wherein the neutralized supernatant is subjected to a dialysis, especially a dialysis against distilled water.
28. Method according to any one of embodiments 21 to 27, wherein separating the neutral polysaccharide from the acidic polysaccharides is performed by anion exchange chromatography on a resin comprising DEAE groups, especially on DEAE cellulose, and in the presence of a buffer, preferably a TRIS/citrate buffer or a phosphate buffer.
29. Method according to any one of embodiments 21 to 28, wherein the incubation of the neutral polysaccharides with endo-β-mannanase is performed at a temperature of 20 to 60°C, preferably at a temperature of 30 to 55°C, especially at a temperature of 40 to 50°C.
30. Method according to any one of embodiments 21 to 29, wherein the incubation of the neutral polysaccharides with endo-β-mannanase is performed for 12 h to 20 d, preferably for 1 d to 10 d, especially for 2 d to 5 d.
31. Prebiotic composition according to any one of embodiments 1 to 20, obtainable by a method according to any one of embodiments 21 to 30.
32. Use of a prebiotic composition according to any one of embodiments 1 to 20, and 31 as a food supplement or functional food.
33. Prebiotic composition according to any one of embodiments 1 to 20, and 31 for use in the prevention or treatment of intestinal disorders, preferably of constipation, increased blood cholesterol levels, distorted balance of intestinal bacterial flora, increased intestinal pH values, , and inflammatory bowel diseases.
34. βGlc-(1→4) [βGal-(1→2)-α-Gal-(1→6) ] Man tetrasaccharide.
35. Nonasaccharide consisting of Glc(1→ ) βMan(1→4)βGlc(1→4) [βGal (1→2)αGal(1→6)]Man hexasaccharide with three βGal monosaccharide residues attached as branches to the hexasaccharide, preferably obtainable by a method according to any one of embodiments 21 to 30.
36. Higher oligosaccharides with a degree of polymerization of 9 - 13, consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide, preferably obtainable by a method according to any one of embodiments 21 to 30.

The present invention is further illustrated by the following examples and the figures, yet without being restricted thereto.
Figure 1. Size-exclusion chromatogram (SEC) of the alkali-soluble polysaccharides from the *Hyptis suaveolens* seed mucilage. Black trace, total polysaccharides; red trace, acid polysaccharide; green trace, neutral polysaccharide.

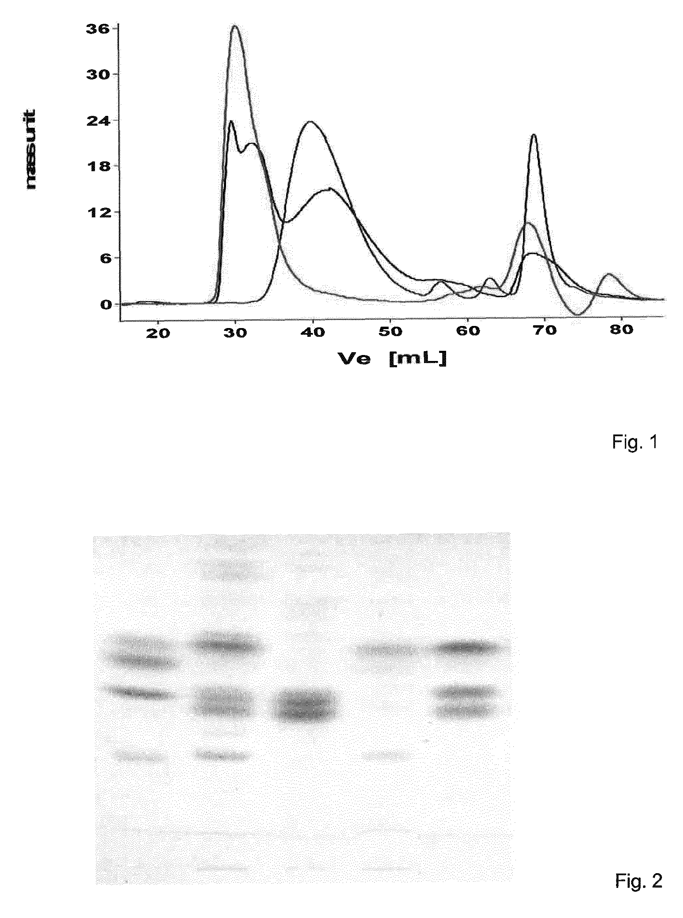

Figure 2. Thin-layer chromatogram (TLC) of the monosaccharide components obtained by hydrolysis (2M trifluoroacetic acid) of the total polysaccharides and separated acid and neutral polysaccharides of *Hyptis suaveolens* together with appropriate standard mixtures. Qualitative monosaccharide compositions of the *Hyptis suaveolens* polysaccharides. Lane 1, mixed standard 1 (from top: fucose, arabinose, glucose, glucuronic acid); lane 2, total alkali-soluble polysaccharides; lane 3, neutral polysaccharide; lane 4, acid polysaccharide; lane 5, mixed standard 2 (from top: xylose, mannose, galactose).
Figure 3. HPAEC (Dionex) chromatogram of a monosaccharide mixture obtained by hydrolysis of the neutral polysaccharide from *Hyptis suaveolens* seed mucilage.
Figure 4. ¹³C-NMR spectrum (125 MHz, D₂O) of the neutral polysaccharide from *Hyptis suaveolens* seed mucilage.
Figure 5. Preparative size-exclusion chromatogram of the hydrolysate reaction mixture obtained by incubation of the neutral polysaccharide from *Hyptis suaveolens* with β-mannanase. For conditions, see Example 4.

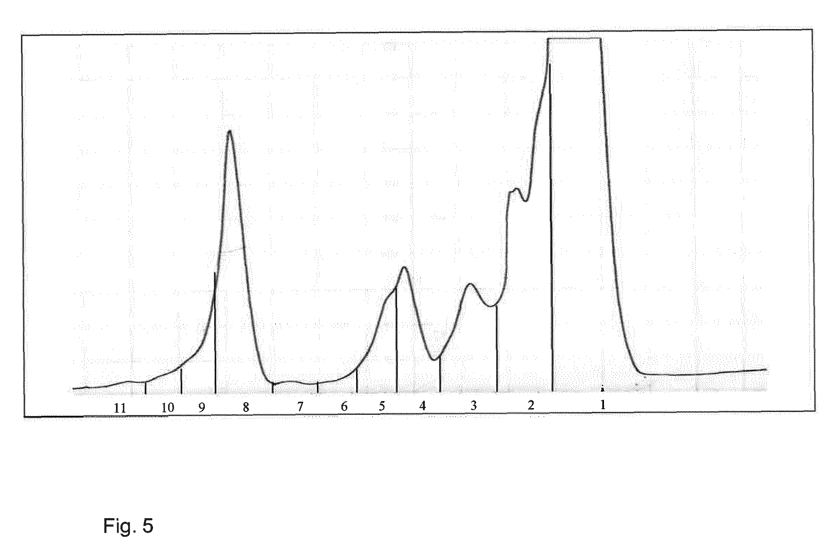

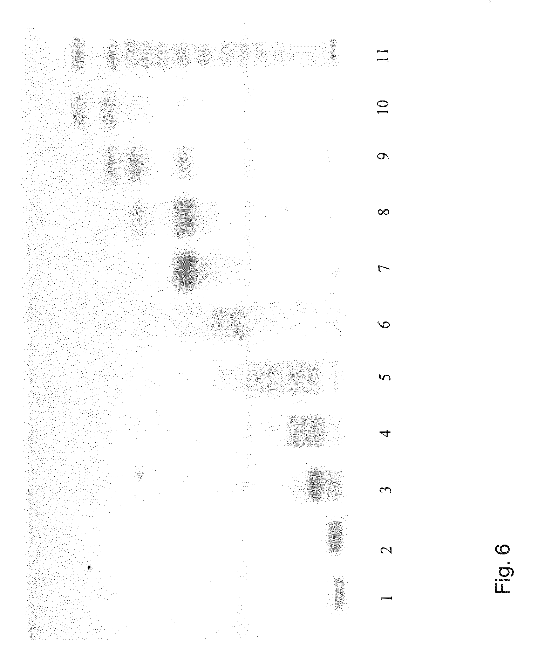

Figure 6. Thin-layer chromatogram of the oligosaccharide fractions obtained by preparative size-exclusion chromatography of the reaction mixture obtained by incubation of the neutral polysaccharide from *Hyptis suaveolens* seed mucilage with β-mannanase. Silica gel Merck 60 F₂₅₄ eluted four times with butanol:propanol:ethanol:water, 2:3:3:2. Lane 1, pool 2 of the SEC represented in Fig. 5. Lane 2, pool 3; lane 3, pool 4; lane 4, pool 5; lane 5, pool 6; lane 6, pool 7; lane 7, pool 8; lane 8, pool 9; lane 9, pool 10; lane 10, pool 11; lane 11, fructooligosaccharides obtained by hydrolysis of inulin from Jerusalem artichoke.
Figure 7. Bioscreen C growth curves obtained with *Lactobacillus reuteri* on MRS minimal medium or MRS minimal medium supplemented with glucose or neutral oligosaccharides from *Hyptis suaveolens* seed mucilage. Full circles, glucose supplement; open circles, small oligosaccharide fraction; open squares, minimal medium without carbohydrate supplementation. For growth conditions, see Example 5.
Figure 8. Bioscreen C growth curves obtained with *Lactobacillus paracasei ssp. paracasei* on MRS minimal medium or MRS minimal medium supplemented with glucose or neutral oligosaccharides from *Hyptis suaveolens* seed mucilage. Full circles, glucose supplement; open circles, small oligosaccharide fraction; open squares, minimal medium without carbohydrate supplementation. For growth conditions, see Example 5.
Figure 9. Bioscreen C growth curves obtained with *Lactobacillus rhamnosus GG* on MRS minimal medium or MRS minimal medium supplemented with glucose or neutral oligosaccharides from *Hyptis suaveolens* seed mucilage. Full circles, glucose supplement; open circles, small oligosaccharide fraction; open squares, minimal medium without carbohydrate supplementation. For growth conditions, see Example 5.
Figure 10. HPAEC (Dionex) chromatogram of a monosaccharide mixture obtained by hydrolysis of the disaccharide βGlc-(1→4)Man.
Figure 11. ¹³C-NMR spectrum (125 MHz, D₂O) of the disaccharide **1** βGlc-(1→4)Man.
Figure 12. ¹H-NMR spectrum (500 MHz, D₂O) of the disaccharide **1** βGlc-(1→4)Man.
Figure 13. HPAEC (Dionex) chromatogram of a monosaccharide mixture obtained by hydrolysis of the trisaccharide **3** βGlc (1→4) [αGal (1→6) ]Man.
Figure 14. ¹³C-NMR spectrum (100 MHz, D₂O) of the trisaccharide **3** βGlc(1→4)[αGal(1→6)]Man.
Figure 15. ¹H-NMR spectrum (400 MHz, D₂O) of the trisaccharide **3** βGlc(1→4)[αGal(1→6)]Man.
Figure 16. HPAEC (Dionex) chromatogram of a monosaccharide mixture obtained by hydrolysis of the tetrasaccharide **4** βGlc-(1→4) [βGal-(1→2)-α-Gal-(1→6) ] Man .
Figure 17. ¹³C-NMR spectrum (100 MHz, D₂O) of the tetrasaccharide **4** βGlc-(1→4) [βGal-(1→2)-α-Gal-(1→6) ] Man.
Figure 18. ¹H-NMR spectrum (400 MHz, D₂O) of the tetrasaccharide **4** βGlc-(1→4) [βGal-(1→2)-α-Gal-(1→6) ]Man.
Figure 19. ¹H-NMR spectrum (500 MHz, D₂O) of a nonasaccharide composed of five galactose, two glucose and two mannose residues.
Figure 20. ¹³C-NMR spectrum (125 MHz, D₂O) of a nonasaccharide composed of five galactose, two glucose and two mannose residues.
Figure 21. HPAEC (Dionex) chromatogram of a monosaccharide mixture obtained by hydrolysis of a nonasaccharide (Lane 3, Pool 4, Figs. 5 and 6) composed of five galactose, two glucose and two mannose residues.
Figure 22. ¹³C-NMR spectrum (125 MHz, D₂O) of a pool of higher oligosaccharides (degree of polymerization, >9 glycosyl residues) obtained by β-mannanase-catalyzed hydrolysis of the neutral polysaccharide from Hyptis suaveolens seeds (Pool 3, Lane 2, Fig.s 5 and 6).
Figure 23. HPAEC (Dionex) chromatogram of the monosaccharide mixture obtained by acid-catalyzed hydrolysis of a pool of higher oligosaccharides (degree of polymerization, >9 glycosyl residues; Pool 3, Lane 2, Figs. 5 and 6). The pool is one of the fractions obtained by β-mannanase-catalyzed hydrolysis of the neutral polysaccharide from Hyptis suaveolens seeds.
Figure 24. Structures of oligosaccharides **1** to **4.**
Figure 25. Structure of oligosaccharide **5.**

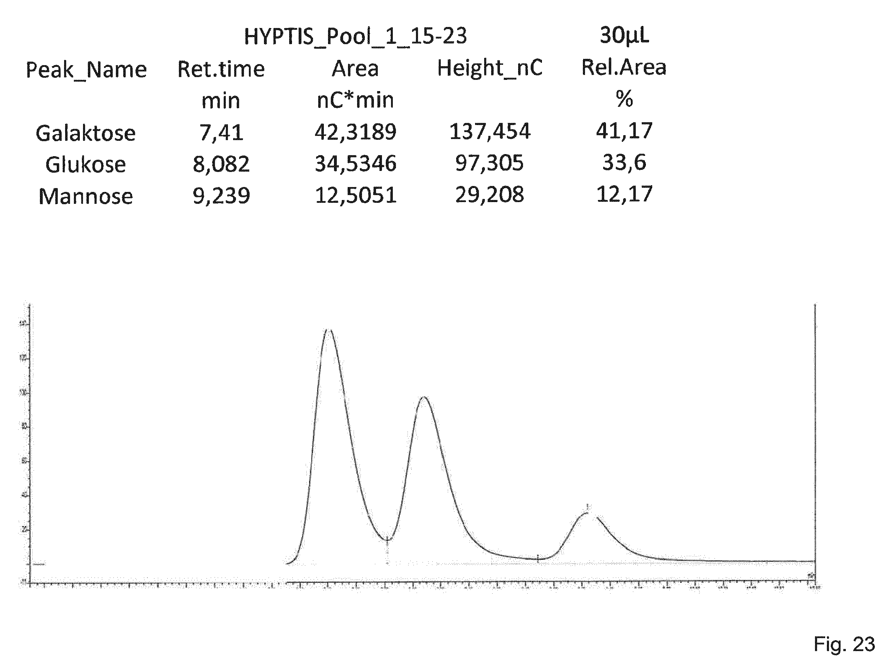

Figure 26. Structural features of a pool of higher oligosaccharides (Pool 3, Lane 2, Figs. 5 and 6, composed of at least two oligosaccharides and represented here by an undecasaccharide) obtained by β-mannanase-catalyzed hydrolysis of the neutral polysaccharide from Hyptis suaveolens. Interglycosidic linkages and anomeric configurations of the additional glucose residues e and f are unknown. The three β-galactose monosaccharide branches are linked to unknown positions on the core mannose and glucose residues c, d, e and f. The ratio of Gal : Glc : Man in this undecasaccharide is 5 : 4 : 2. According to the HPAEC (Dionex) chromatogram shown in Fig. 23, the monosaccharide ratio of Pool 3, Lane 2, Fig.s 5 and 6 is ca. 5 : 4.1 : 1.6.
Figure 27 shows structures of the model compounds methyl β-D-galactopyranoside **7,** lactose **8** and lactitol **9.**
Figures 28-30 show ¹³C-NMR spectra of structures **7, 8** and **9.**

### Examples:

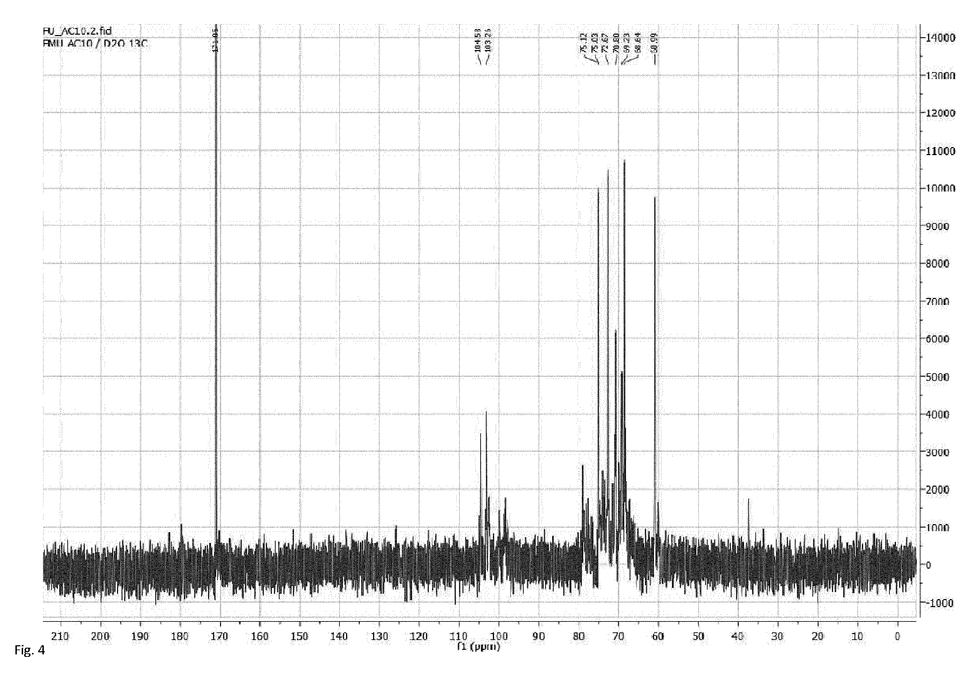

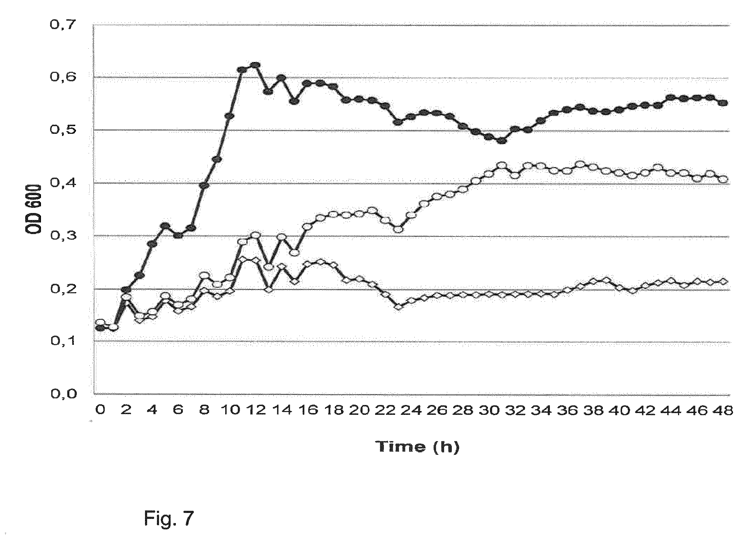

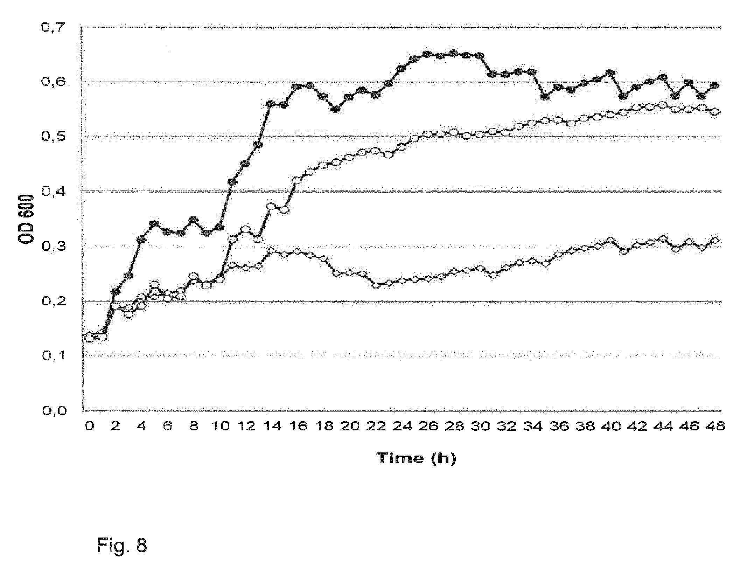

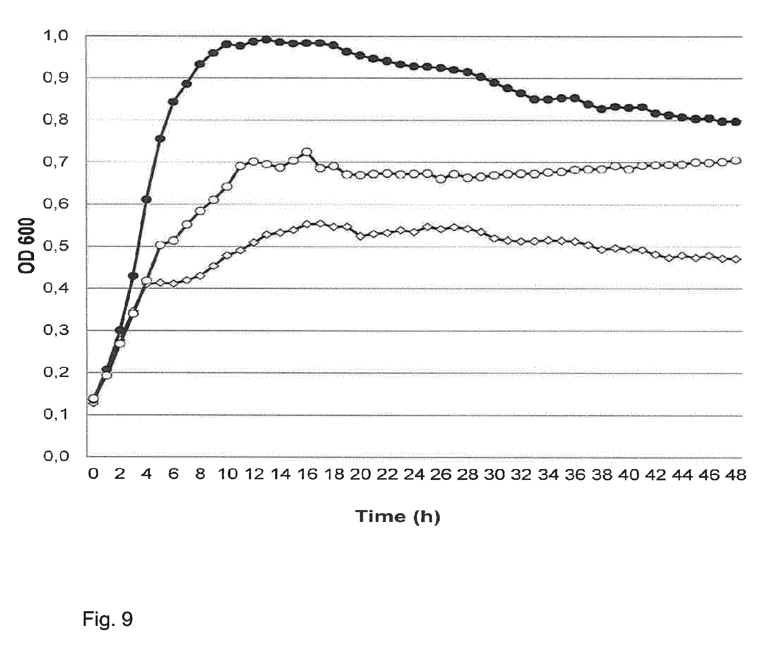

The prebiotic oligosaccharides according to the present invention (composed of glucose, galactose and mannose) can be prepared from the neutral polysaccharide of *Hyptis suaveolens* seeds. Aqueous workup of *H. suaveolens* seeds provides a mucilage composed mostly of polysaccharides and other components such as proteins.
- The mucilage is first submitted to treatment with alkali to remove esterified components and proteins.
- The alkali-treated mucilage is a 1:1 mixture of an acidic and a neutral polysaccharide; it is separated into the acidic and neutral polysaccharides by column chromatography over an anion exchange resin (Fig. 1).
- By monosaccharide analysis (acid-catalyzed hydrolysis and thin layer chromatography, TLC), the acidic polysaccharide contains xylose, fucose and glucuronic/galacturonic acids whereas the neutral polysaccharide contains glucose, galactose and mannose (Fig. 2). In the ¹³C-NMR spectrum, individual carbon resonances are widely scattered as expected for this type of irregular heteropolysaccharide. However, the characteristic pattern attributed to C-2, C-3, C-4, and C-5 of a nonreducing-terminal, β-galactopyranosyl residue clearly stands out and exceeds all other resonance signals in intensity (Fig. 4). This indicates that the majority of galactose residues is present in the form of nonreducing β-galactopyranose monosaccharide side chains ("stubs") attached to various positions along the glucomannan backbone.
- The neutral polysaccharide is incubated with *endo*-β-mannanase to provide a mixture of oligosaccharides that can be separated by preparative size exclusion chromatography (SEC; Fig. 5). Pools of the fractions obtained by SEC are characterized by TLC (Fig. 6).
- Pools of either the higher or the lower oligosaccharides, or total pools of higher and lower oligosaccharides, when added to suitable bacterial cultures in minimal media support the growth of probiotic bacteria such as *Lactobacillus rhamnosus* GG, *Lactobacillus reuteri,* or *Lactobacillus paracasei* (Figs. 7, 8 and 9).
- The structures of several oligosaccharides from the seed mucilage of *Hyptis suaveolens* have been characterized by high pH-anion exchange chromatography with pulsed amperometric detection (HPAEC-PAD), ¹H- and ¹³C-nuclear magnetic resonance (NMR)-spectroscopy.

### Example 1. Preparation of the alkali-soluble polysaccharide fraction from Hyptis suaveolens seed mucilage.

By treatment with alkali, water- or alkali-soluble polysaccharides can be separated from accompanying materials such as proteins. Mucilage from *Hyptis suaveolens* seeds (5.0 g) was suspended in 4 M NaOH (150 mL) in a 500 mL Erlenmeyer flask. The suspension was covered with parafilm and was magnetically stirred for 48 h at 60°C. Subsequently, distilled water (100 mL) was added and the suspension centrifuged for 10 min at 10 000 rpm. The supernatant was drawn off and the insoluble residue washed twice with 2 M NaOH (100 mL each) and centrifuged. The supernatants were combined (450 mL, pH ca. 13.9) and were neutralized with 25% hydrochloric acid in an ice bath with magnetic stirring. The final volume is ca. 600 mL. For dialysis, the sample solution was distributed into three dialysis tubes (200 mL each) and dialyzed against distilled water for 3 days. To prevent microbial growth, a small amount of sodium azide was added to the sample solutions. Subsequently, the inner dialyzate was shell-frozen in 500 mL round-bottomed flasks (ca. 150 mL per flask). The solutions were lyophilized over night using a HETO-PowerDry lyophilizer. In this manner, a yield of 2.77 g (ca. 55%) of alkali-soluble polysaccharide is obtained from crude *Hyptis suaveolens* mucilage. Fig. 1 shows the size-exclusion chromatograms (SEC) of the alkali-soluble polysaccharides from *Hyptis suaveolens* (black trace, total polysaccharides).

### Example 2. Separation of the alkali-treated Hyptis suaveolens mucilage into acidic and neutral polysaccharides.

DEAE-Cellulose (Whatman DE52, 37.2 g) was equilibrated with 0.01 M Tris.citrate buffer and placed in a column (2.5 × 30 cm). Alkali-soluble polysaccharide mixture of *Hyptis suaveolens* (170 mg, see Example 1) was dissolved in 0.01 M Tris.citrate buffer (5 mL) and applied to the anion-exchange column. The column was rinsed with 0.01 M Tris.citrate buffer, and fractions (10-12 mL) were collected at a flow rate of 1 mL/min. To examine for the presence of polysaccharide in the fractions, a drop from each fraction was spotted onto a narrow strip of tlc foil (silica gel) and sprayed with thymol reagent. The spray reagent is prepared by dissolving 0.5 g thymol in 95% ethanol and carefully adding 5 mL of 97% sulfuric acid. Color is developed by heating for 15-20 min at 120°C. Neutral polysaccharide was found in fractions 2-6. Subsequently, acid polysaccharide was eluted with 0.5 M sodium chloride in 0.01M Tris-citrate pH = 7.5 (fractions 11-15). Fig. 1 shows the SEC of the separated acid and neutral polysaccharides from *Hyptis suaveolens* (red trace, acid polysaccharide; green trace, neutral polysaccharide).

### Example 3. Qualitative monosaccharide compositions of the alkali-soluble (total) polysaccharides and of the separated acid and neutral polysaccharides from Hyptis suaveolens. Quantitative composition of the neutral polysaccharide.

For analysis of the monosaccharide composition, the polysaccharide sample (2 mg) was suspended in 2M trifluoroacetic acid (1 mL) in a Reacti-Vap pressure vessel equipped with a stirring magnet. The sealed vessel was heated with stirring for 2 h at 100°C. Subsequently, the vessel was allowed to cool to room temperature, methanol (1 mL) was added and the volatiles (including trifluoroacetic acid) removed by passing through a gentle stream of nitrogen at 50°C under a well-vented fume hood. For the complete removal of trifluoroacetic acid, 10 additions/evaporations with 1 mL each of methanol were performed. For analysis, the dried samples were dissolved in methanol (1 mL). For thin layer chromatography (TLC), samples were applied manually or automatically (CAMAG Linomat 5) on silica gel plates (10 × 10 cm) and developed in acetonitrile:water 17:3. For optimal separation, chromatograms were developed 2-4 times in the same eluent. Fig. 2 shows a TLC (silica gel 60 F254, acetonitrile:water, 17:3) of the monosaccharide components obtained by hydrolysis (2M TFA) of the total polysaccharides and separated acid and neutral polysaccharides of *Hyptis suaveolens* together with appropriate standard mixtures.

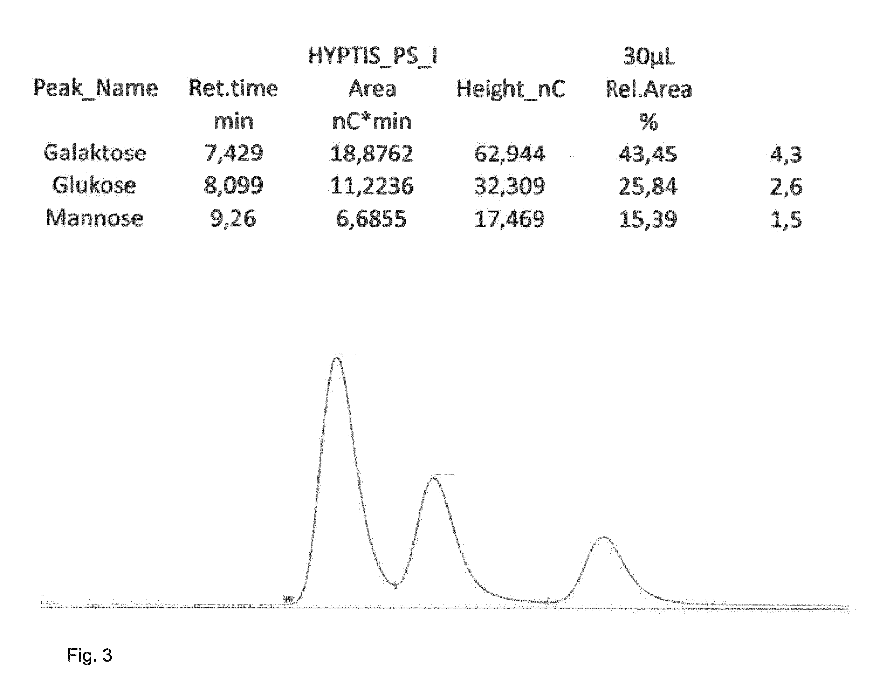

The quantitative monosaccharide composition of the neutral polysaccharide was determined by analytical high-performance anion exchange chromatography with pulsed amperometric detection (HPAEC-PAD). This method takes advantage of the weakly acidic nature of carbohydrates to give selective separations at high pH using a strong anion-exchange stationary phase [Hardy MR, Townsend RR, Lee YC Anal. Biochem. 170, 54 - 62 (1988)]. Using HPAEC-PAD, the molar composition of the neutral polysaccharide was determined as galactose : glucose : mannose, ca. 8.6 : 5.2 : 3 (Fig. 3). From the ¹³C-NMR spectrum (Fig. 4), it may be concluded that the majority of the galactose units are present as nonreducing β-galactopyranosyl monosaccharide "stubs".

### Example 4. Cleavage with endo-β-mannanase of the neutral polysaccharide from Hyptis suaveolens. Preparative size exclusion chromatography.

Neutral polysaccharide of *Hyptis suaveolens* (250 mg, see Example 3 above) was dissolved in doubly distilled water (3 ml) at 65°C over night. Purified endo-β-1,4-mannanase (500 µl) was added and the whole incubated at 45°C for 3 d. Subsequently, the solution was briefly heated to 100°C, the denatured enzyme protein was removed by centrifugation, and the solution lyophilized for preparative size-exclusion chromatography.

The lyophilizate (ca. 50 mg) was dissolved in doubly distilled water (2 ml) and applied to a sequence of two columns (column 1, Biogel P2, 90×1.5 cm; column 2, Biogel P4, 130×1.5 cm) at a flow rate of 0.2 ml/min. Carbohydrate content of the eluate was measured with the aid of a differential refractometer and fractions (5 ml) were collected in a fraction collector. As illustrated in Figure 5, fractions were combined into 11 pools (numbered 1 - 11) and lyophilized. Pools were analyzed by thin layer chromatography as shown in Figure 6.

### Example 5. In vitro growth curves of common probiotic bacterial strains on minimal medium supplemented with oligosaccharide fractions from the neutral polysaccharide of Hyptis suaveolens.

Growth enhancement of lactobacillus strains with oligosaccharide pools obtained by β-mannanase catalyzed hydrolysis of the neutral polysaccharide from *Hyptis suaveolens.* - Bacterial cells (300 µl) were seeded into 100 well microplates at an optical density (OD 600) of 0.1. The growth medium was MRS (deMan, Rogosa and Sharpe) minimal medium containing 10 g peptone from casein, 8 g meat extract, 4 g yeast extract, 0.5 g L-cysteine hydrochloride, 2 g potassium phosphate dibasic, 1 g Tween 80, 2 g ammonium citrate dibasic, 5 g sodium acetate, 0.2 g magnesium sulfate, and 0.04 g manganese sulfate. Bacteria were cultivated in the microplates in the presence or absence of 0.5 % sugar or sample at 37°C for 48 h using the Bioscreen C incubator/reader system. The turbidity (OD 600) was measured every hour and growth curves were derived. Growth curves obtained with Lactobacillus reuteri, Lactobacillus paracasei ssp. paracasei and Lactobacillus rhamnosus GG are shown in Figs. 7, 8 and 9.

### Example 6. Oligosaccharide part structures of the neutral polysaccharide from Hyptis suaveolens seed mucilage.

HPAEC and NMR analyses were performed of disaccharide 1 and trisaccharide **3** formed by incubation of the neutral polysaccharide from *Hyptis suaveolens* with a crude preparation of β-galactosidase from *Aspergillus* containing small amounts of α-galactosidase and β-mannanase activity.

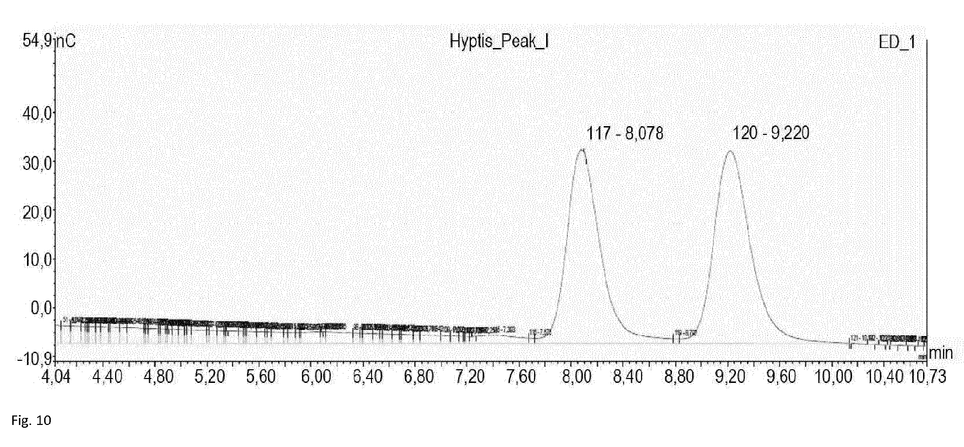

Fig. 10 shows a HPAE (Dionex) chromatogram of a monosaccharide mixture obtained by hydrolysis of the disaccharide βGlc-(1→4)Man **(1** in Plate 1), demonstrating the ratio of glucose to mannose as 1:1.

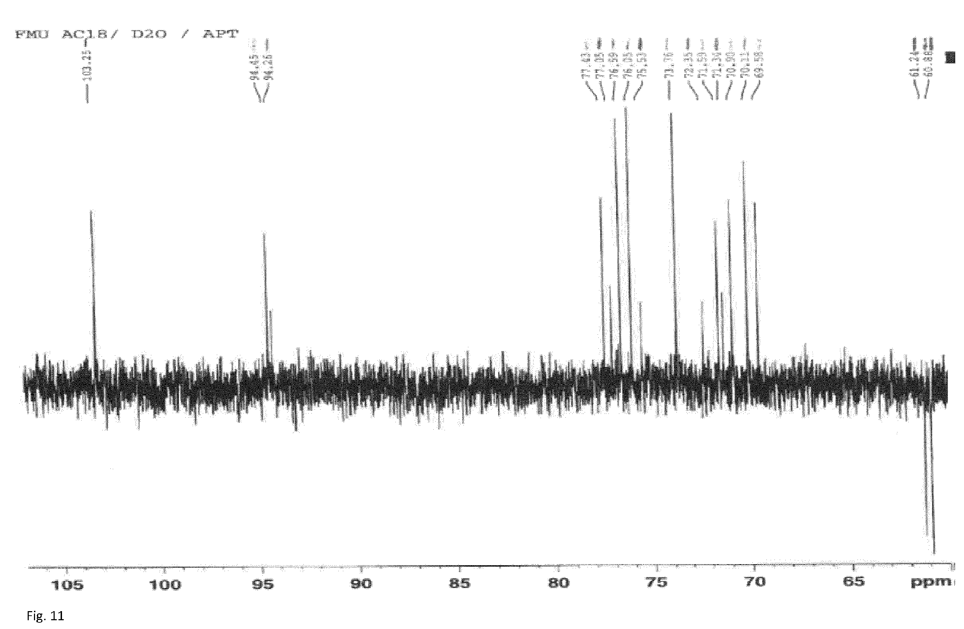

In Fig. 11, the ¹³C-NMR-spectrum is shown of **1** (125 MHz, D₂O). In the anomeric region, signals are at 103.3 (C-1 βGlc), 94.5 (C-1 αMan) and 94.3 ppm (C-1 βMan). In the region of secondary carbon atoms (δ 69.6 - 77.4 ppm), there are 12 signals as expected (C-2,3,4,5 of the βGlc, αMan and βMan residues). The signals at 76.6 (C-5 βGlc), 76.1 (C-3 βGlc), 73.8 (C-2 βGlc) and 70.1 ppm (C-4 βGlc) are attributed to the nonreducing β glucose unit, as is the signal for C-6 at 61.2 ppm.

The signals attributable to the reducing Man unit are 77.4 (C-4; glycosylation shift by 10 ppm: the C-4 resonance in methyl α-D-mannopyranoside is at 67.4 ppm), 71.6 (C-5), 70.9 (C-2) and 69.6 ppm (α-anomer), and 77.1, 75.5, 72.4 and 71.3 ppm (β-anomer). The signal for C-6 of the reducing Man unit is at 60.9 ppm. This pattern is closely similar to that of the reducing mannose unit in the standard β-1,4-linked mannotriose **(2** in Fig. 24), where the resonances attributable to the C-2, 3, 4, and 5 of the reducing mannose residue are at 77.4, 71.5, 70.8 and 69.5 ppm (α-anomer) and 77.2, 75.4, 72.3 and 71.2 ppm (β-anomer). The signal for C-6 is at 61.1 ppm.

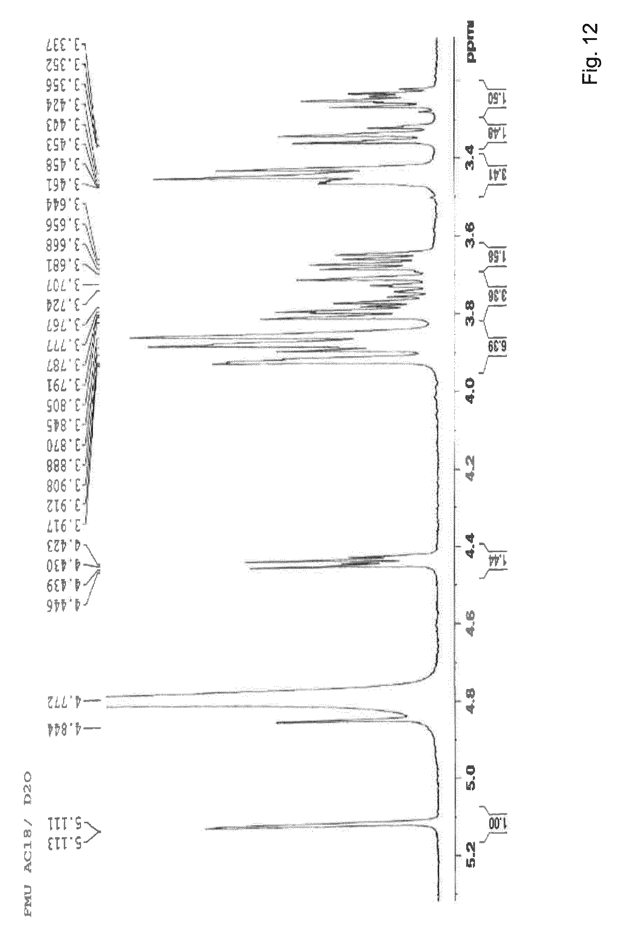

The ¹H-NMR-spectrum of **1** (500 MHz, D₂O) is shown in Fig. 12 and is only partially interpretable. In the region of anomeric resonances (δ 4.4 - 5.2 ppm), the expected signals are observed for H-1 of the reducing αMan unit (5.11), H-1 of the reducing βMan (4.84) and for H-1 of the β-glucopyranosyl unit (4.43 ppm). Disaccharide **1** (4-[β-D-glucopyranosyl]-D-mannose is an epimer of cellobiose and is known in the literature as epicellobiose. It has been synthesized (M. Bergmann and H. Schotte, Ber. Dtsch. Chem. Ges. 54, 1564, 1921; W. T. Haskins, R. M. Hann and C. S. Hudson, J. Amer. Chem. Soc., 63, 1724, 1941).

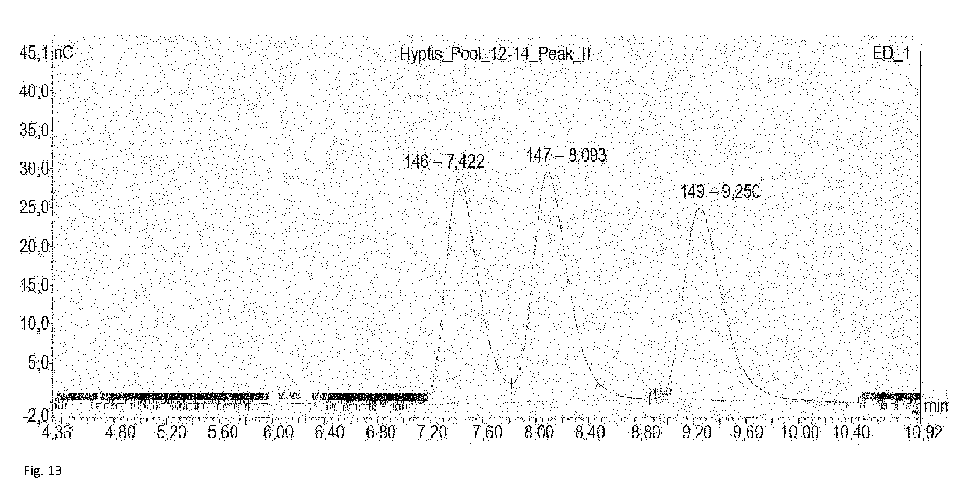

Fig. 13 shows a HPAE (Dionex) chromatogram of a monosaccharide mixture obtained by hydrolysis of the trisaccharide βGlc-(1→4)[αGal-(1→6)]Man **(3** in Fig. 24), demonstrating the ratio of galactose: glucose: mannose to be 1:1:1.

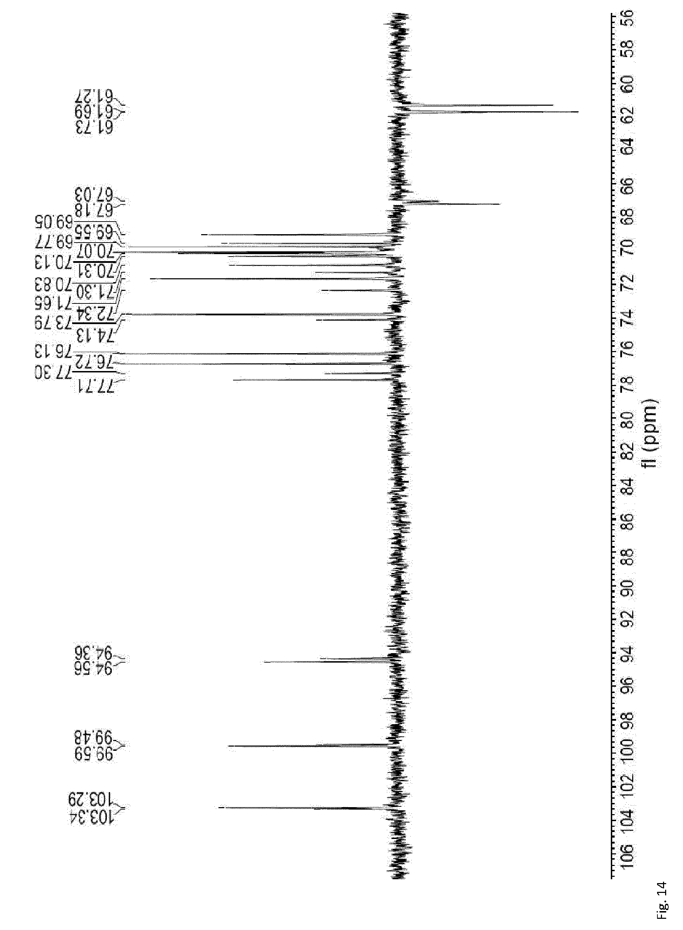

The ¹³C-NMR spectrum of trisaccharide **3** is shown in Fig. 14. In the anomeric region, the expected four signals are observed at δ 103.3 (C-1 of βGlc), 99.6 (C-1 of αGal), 94.6 (C-1 of αMan) and 94.4 (C-1 of βMan). Both the glucose and galactose C-1 signals show slight splitting due to the anomery of the mannose residue. In the region of secondary carbon atoms (δ 69 - 78 ppm), a total of 16 lines are observed, corresponding to the C-2,3,4,5 of the βGlc, αGal, and α and βMan residues. The βGlc unit shows resonances at δ 76.7 (C-5), 76.1 (C-3), 73.8 (C-2) and 70.1 ppm (C-4), practically identical with the signals of the equivalent residue in disaccharide **1.** Attibutable to αGal are the resonances at δ 71.7 (C-5), 70.1 (C-3), 69.8 (C-4) and 69.1 ppm (C-2). This latter signal shows splitting due to the anomery of the mannose unit, to which αGal is attached in a (1→6)-linkage. Resonances attributable to the α-anomer of the mannose unit are at 77.7 (C-4; glycosylation shift as in **1),** 70.8 (C-5; β-shift of 0.8 ppm due to substitution at C-6), 70.3 (C-3) and 69.6 ppm (C-2). Resonances for the β-anomer of the reducing Man unit are at 77.3, 74.1, 72.4 and 71.3 ppm. Significantly, the C-6 resonances of the Man unit are glycosylation shifted by ca. 6 ppm (α, δ 67.2, β, 67.0 ppm; the C-6 resonance in methyl α-D-mannopyranoside is at δ 61.6 ppm).

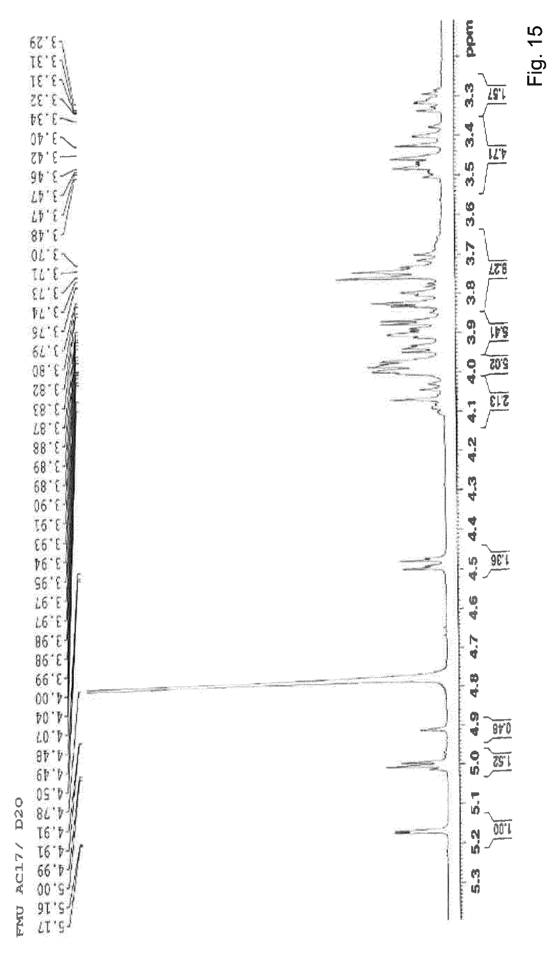

The ¹H-NMR spectrum of trisaccharide **3** (Fig. 15) shows significant analogy with that of disaccharide **1** in the anomeric region (δ 4.4 - 5.2 ppm). Signals due to the H-1 of the α- and β-mannopyranosyl units (δ 5.16 and 4.91 ppm) are in close proximity to the equivalent signals in disaccharide **1** (5.11 and 4.84 ppm). The signal for H-1 of the glucose unit is at 4.49 (H-1 Glc in **1,** 4.43 ppm). The additional signal at 4.99 ppm is attributable to the H-1 of αGal: in raffinose [αGal-(1→6)-αGlc-(1→2)Fru], the resonance for H-1 of the nonreducing terminal αGal residue is at δ 4.98 ppm, J_{1,2}∼ 3.7 Hz).

### Example 7. HPAEC and NMR data of higher oligosaccharide part structures of the neutral polysaccharide from Hyptis suaveolens. NMR spectra of a tetra- and a nonasaccharide.

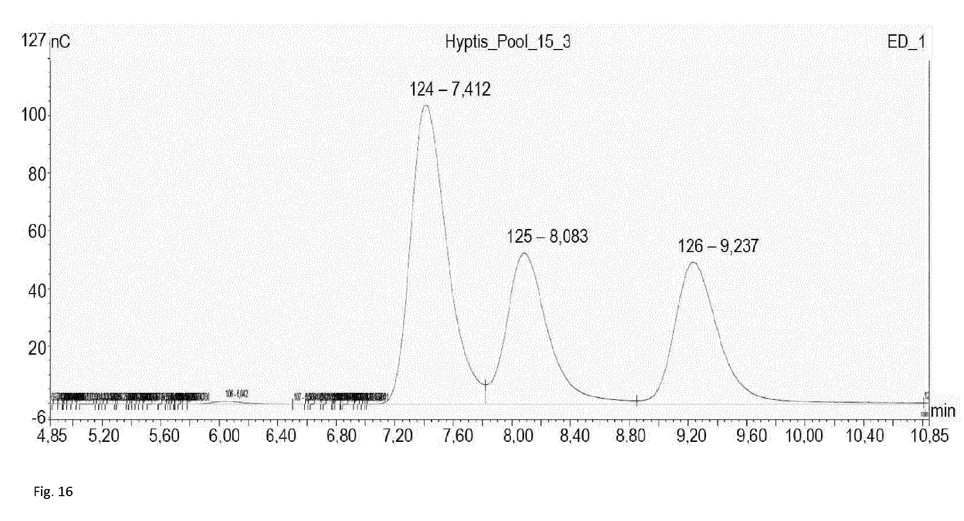

Tetra- and nonasaccharide part structures of the neutral polysaccharide from *Hyptis suaveolens* seed mucilage were structurally characterized by HPAEC-PAD, ¹H- and ¹³C-NMR spectroscopy. Fig. 16 shows a HPAE (Dionex) chromatogram of a monosaccharide mixture obtained by hydrolysis of the tetrasaccharide **4** (Plate 1) tentatively identified as βGlc-(1→4)-[βGal-(1→2)-αGal-(1→6)]-Man; the chromatogram proves the monosaccharide composition to be galactose: glucose: mannose, 2:1:1.

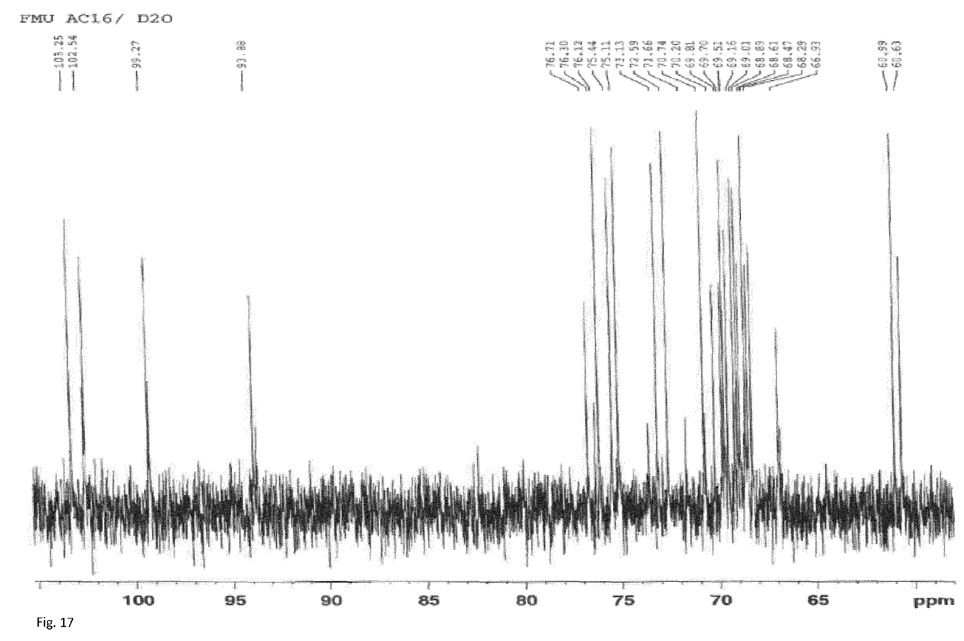

The ¹³C-NMR spectrum of tetrasaccharide **4** is shown in Fig. 17. In the anomeric region, five signals are recorded. These are at δ 103.3 ppm (C-1 of βGal; the signal shows no splitting and is therefore the farthest removed from the reducing mannose residue), 102.5 (C-1 of βGlc), 99.3 (C-1 of αGal), 93.9 (C-1 of reducing αMan) and 93.7 ppm (C-1 of reducing βMan). In the region corresponding to the secondary ring carbon atoms (C-2 -C-5), there are 21 lines corresponding to five pyranose residues plus one carbon atom; the additional signal is considered due to splitting consequent to the anomery of the mannose unit. Twelve lines are more intense than the rest and presumably can be attributed to the glycosidically linked αGal, βGal, and βGlc units, while the remaining lines are due to the α- and β-reducing Man units. In the region of primary alcohol groups (C-6 of hexopyranose residues) there are five lines as expected, 61.3 (C-6 αGal), 61.7 (2×, C-6 βGlc βGal), 67.5 (C-6 βMan) and 67.6 ppm (C-6 αMan; the Man signals are glycosylation shifted as in **3**).

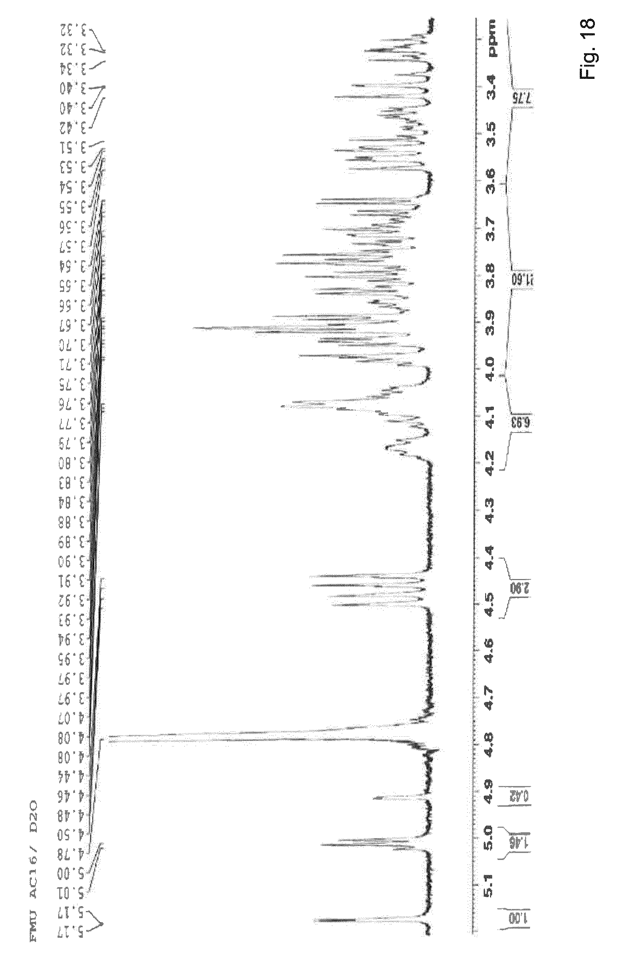

The anomeric region of the ¹H-NMR spectrum of tetrasaccharide **4** (Fig. 18) is analogous to the corresponding regions in disaccharide **1** and trisaccharide **3,** except for the addition of a signal due to H-1 of a β-galactose residue. Signals are at δ 5.17 (H-1 of reducing α-Man), 5.00 (H-1 of αGal), 4.91 (H-1 of reducing βMan), 4.49 (H-1 of βGlc) and 4.47 ppm (H-1 of βGal).

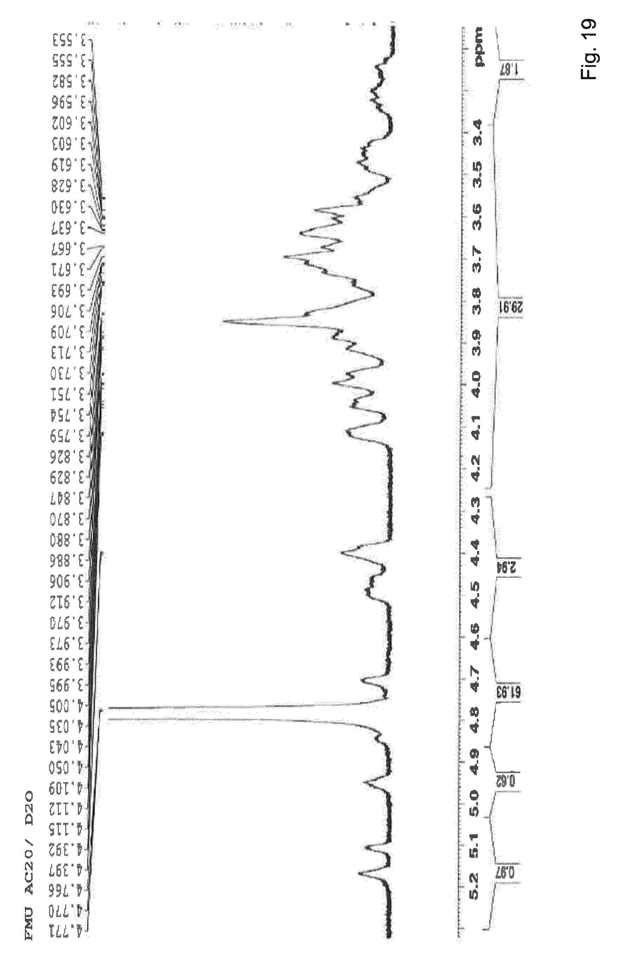

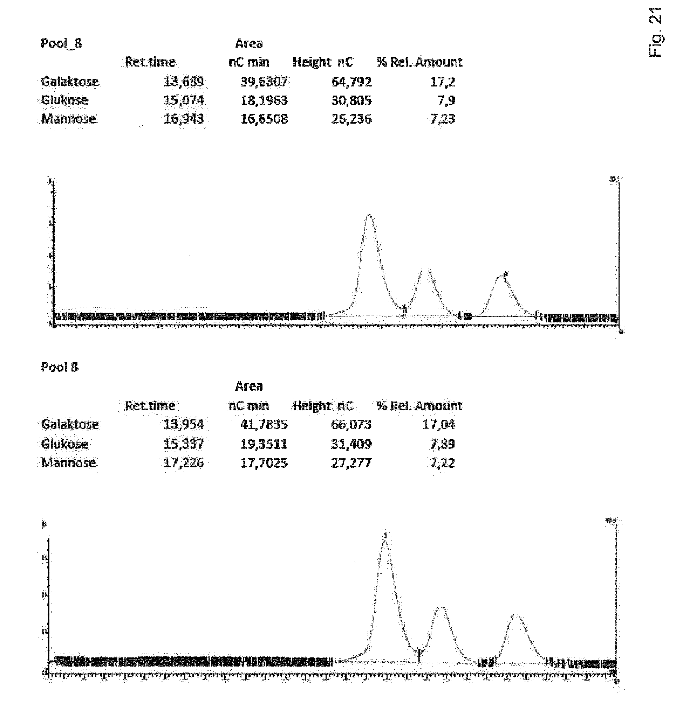

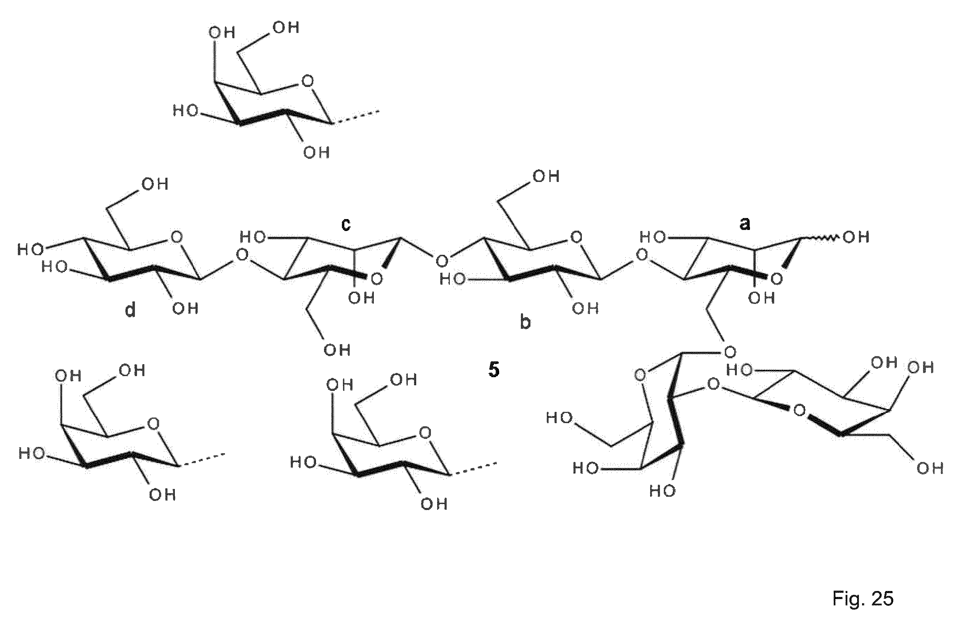

The ¹H-NMR spectrum of a nonasaccharide, presumably of structure **5** (Fig. 25; Pool 4, lane 3 in Figs. 5 and 6) is shown in Fig. 19. Spectral assignments concerning **5** are supported by the results of monosaccharide analysis (HPAEC-PAD, Fig. 21, indicating a ratio of Gal : Glc : Man 5 : 2 : 2) and are subject to verification by enzymatic analysis, methylation analysis and additional NMR-spectroscopic experiments. In the region of anomeric protons, there are signals corresponding to ten hydrogen atoms, namely δ 5.18 (H-1 of glycosidically linked α-Glc), 5.11 (H-1 of reducing α-Man), 4.95 (H-1 of αGal), 4.85 (H-1 of reducing βMan), 4.71 (H-1 of glycosidically linked βMan), 4.50, 4.47, and 4.41 ppm (5 × H-1 of βGal/βGlc units). As in oligosaccharides **1, 3** and **4,** the integrals of the signals due to the anomers of reducing Man add up to one proton, so that the presence of a reducing Man unit in **5** is confirmed. Furthermore, the data indicate that the reducing portion of **5** is analogous to tetrasaccharide **4.** Presumably, the additional βMan unit in **5** is an endo-β-mannanase cleavage site which would give rise to a pentasaccharide in addition to tetrasaccharide **4.** This kind of oligosaccharide might be contained in one of the pools of the preparative SEC shown in Fig. 6. However, identification must await the availability of sufficient amounts of the respective cleavage products.

The ¹³C-NMR spectrum of **5** (D₂O, 125 MHz, Fig. 20) shows six prominent signals at δ 103.9 (C-1 of βGal), 75.7 (C-5 of βGal), 73.3 (C-3 of βGal), 71.4 (C-2 of βGal), 69.2 (C-4 of βGal) and 61.6 ppm (C-6 of βGal). This group of signals is attributable to three identical βGal units attached as side branches in unknown positions to the main chain as represented in structure **5,** Fig. 25.

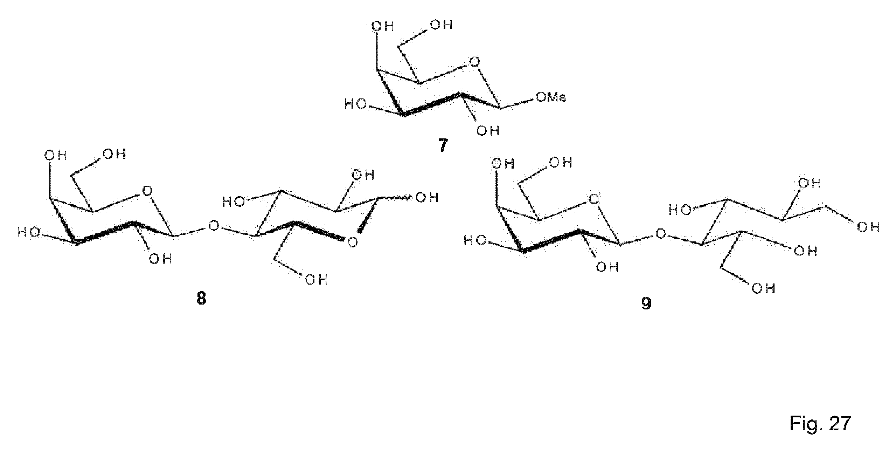

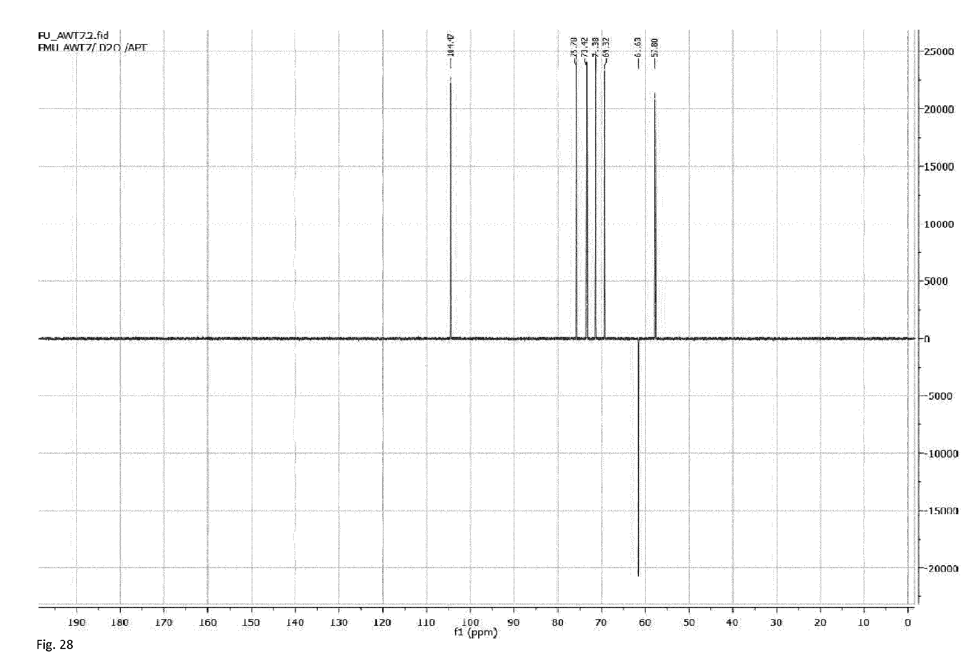

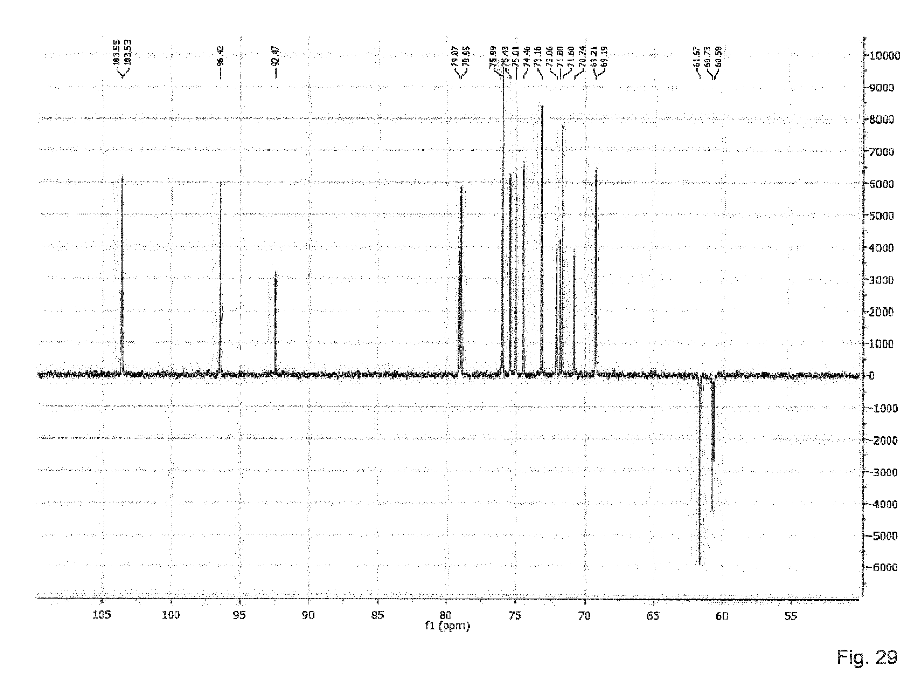

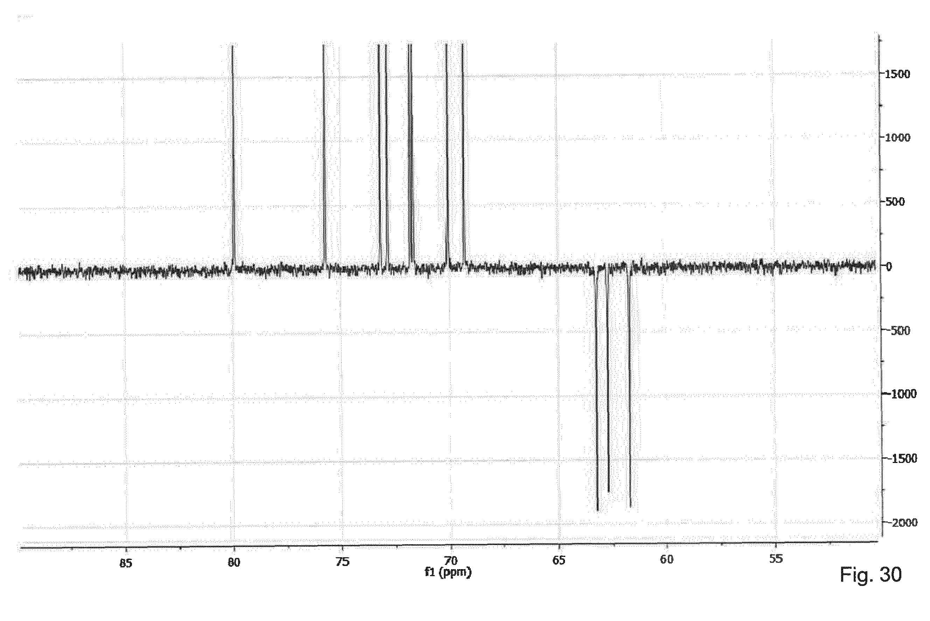

The four characteristic signals attributable to carbon atoms 5, 3, 2 and 4 of the β-galactopyranosyl unit are also seen in the ¹³C-NMR spectrum of the neutral polysaccharide (Fig. 4), the tetrasaccharide **4** and of the model compounds methyl β-D-galactopyranoside **(7),** lactose **(8)** and lactitol **(9;** Table 1, Fig. 27 and Figs. 28-30).

**Table 1: ¹³C chemical shifts attributed to C-2, 3, 4 and 5 of β-D-galactopyranosyl residues in Hyptis neutral polysaccharide, oligosaccharides and model compounds.**

| | | oligosaccharides | | | model compounds | | |
|---|---|---|---|---|---|---|---|
| C | NPS | **4** | **5** | **6** | **7** | **8** | **9** |
| 5 | 75.8 | 75.8 | 75.7 | 75.7 | 75.8 | 76.0 | 75.7 |
| 3 | 73.3 | 73.3 | 73.3 | 73.2 | 73.4 | 73.2 | 73.2 |
| 2 | 71.6 | 71.4 | 71.4 | 71.4 | 71.4 | 71.6 | 71.7 |
| 4 | 69.3 | 69.3 | 69.2 | 69.2 | 69.3 | 69.2 | 69.3 |

A similar group of signals is present in the ¹³C-NMR spectrum (Fig. 22) of a pool of higher oligosaccharides of degree of polymerization >9 glycosyl residues with the ratio of Gal : Glc : Man ca. 5 : 4.1 : 1.6 (structure **6,** Fig.26; analyzed by HPAEC-PAD, Fig. 23; Pool 3, Lane 2, Figs. 5 and 6). In this pool, the proportion of glucose relative to galactose and mannose is higher than in the native polysaccharide, indicating that the composition of the polysaccharide core is heterogeneous and contains regions where the content of glucose outweighs the content of mannose more than twofold. Such regions would contain fewer β-mannanase cleavage sites, so that β-mannanase-catalyzed hydrolysis would give rise to larger oligosaccharides.

### References

D. C. Gowda, Polysaccharide components of the seed-coat mucilage from Hyptis suaveolens, Phytochemistry, 23, 337 - 338 (1984).
G. O. Aspinall, P. Capek, R. C. Carpenter, D. C. Gowda, and J. Szafranek, A novel L-fuco-4-O-methyl-D-glucorono-D-xylan from Hyptis suaveolens, Carbohydrate Research, 214, 107 - 113 (1991).
S. Schorn, Enzymkatalysierte Spaltung der Polysaccharide aus den Samen von Hyptis suaveolens (L.) Poit, Diplomarbeit Universität Wien, Fakultät fur Lebenswissenschaften, 2009.

## Claims

1. Prebiotic composition comprising one or more oligosaccharides, selected from the group consisting of
- a βGlc-(1→4) [βGal-(1→2)-α-Gal-(1→6) ] Man tetrasaccharide,
- a nonasaccharide consisting of a Glc(1→ ) βMan(1→4)βGlc(1→4)[βGal (1→2)αGal(1→6)]Man hexasaccharide with three βGal monosaccharide residues attached as branches to the hexasaccharide, and
- higher oligosaccharides with a degree of polymerization 9 - 13 consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide,
wherein the tetrasaccharide and/or the nonasaccharide and/or the higher oligosaccharides is/are contained in the composition in an amount of at least 5 % w/w, preferably at least 10 % w/w, especially at least 25 % w/w, dry weight.

2. Prebiotic composition according to claim 1, further comprising a βGlc-(1→4)-[αGal-(1→6) ] Man trisaccharide, preferably in an amount of at least 5 % w/w, more preferred at least 10 % w/w, especially at least 25 % w/w, dry weight.

3. Prebiotic composition according to claim 1 or 2, further comprising a βGlc-(1→4)Man disaccharide, preferably in an amount of at least 5 % w/w, more preferred at least 10 % w/w, especially at least 25 % w/w, dry weight.

4. Prebiotic composition according to any one of claims 1 to 3, comprising a hydrolysate of one or more heteropolysaccharides from plants, especially from plant seeds.

5. Prebiotic composition according to any one of claims 1 to 4, comprising a hydrolysate of one or more neutral heteropolysaccharides from plant seeds of *Hyptis suaveolens.*

6. Prebiotic composition according to any one of claims 1 to 5, having a ratio Gal : Glc : Man from 2 : 1 : 1 to 3.2 : 2.6 : 1, especially of about 9 : 5 : 3 or 2.5 : 1 : 1.

7. Prebiotic composition according to any one of claims 1 to 6, wherein the composition is obtained from mucilage of *Hyptis suaveolens* seeds.

8. Method for producing a prebiotic composition comprising the following steps:
- providing a mucilage of *Hyptis suaveolens* seeds;
- treatment of the mucilage with an alkaline agent, preferably a KOH or NaOH containing agent, thereby obtaining a supernatant and an insoluble part;
- separating the supernatant from the insoluble part, preferably by centrifugation, wherein the supernatant contains acidic and neutral polysaccharides;
- neutralizing the supernatant, preferably to a pH of 6 to 8;
- separating the neutral polysaccharide from the acidic polysaccharides, preferably by a chromatographic method, especially by use of an anion exchange resin, thereby obtaining the neutral polysaccharides;
- incubation of the neutral polysaccharides with *endo*-β-mannanase, thereby obtaining a composition comprising oligosaccharides.

9. Method according to claim 8, wherein the composition comprising oligosaccharides obtained contains one or more oligosaccharides, selected from the group consisting of
- a βGlc-(1→4) [βGal-(1→2)-α-Gal-(1→6) ] Man tetrasaccharide,
- a βGlc-(1→4)-[αGal-(1→6)]Man trisaccharide
- a nonasaccharide consisting of a Glc(1→ ) βMan(1→4)βGlc(1→4)[βGal (1→2)αGal(1→6)]Man hexasaccharide with three βGal monosaccharide residues attached as branches to the hexasaccharide, and
- higher oligosaccharides with a degree of polymerization 9 - 13 consisting of a Glc(1→ ) Glc(1→ ) Glc(1→ ) βMan (1→4) βGlc (1→4) [βGal (1→2) αGal (1→6) ] Man with three or more β-Gal monosaccharide residues attached as branches to the octasaccharide.

10. Prebiotic composition according to any one of claims 1 to 20, obtainable by a method according to claim 8 or 9.

11. Use of a prebiotic composition according to any one of claims 1 to 7 as a food supplement or functional food.

12. Prebiotic composition according to any one of claims 1 to 7 for use in the prevention or treatment of intestinal disorders, preferably of constipation, increased blood cholesterol levels, distorted balance of intestinal bacterial flora, increased intestinal pH values, and inflammatory bowel diseases.

13. βGlc-(1→4) [βGal-(1→2)-α-Gal-(1→6) ] Man tetrasaccharide.

14. Nonasaccharide consisting of Glc(1→ ) βMan(1→4)βGlc(1→4)[βGal (1→2)αGal(1→6)]Man hexasaccharide with three βGal monosaccharide residues attached as branches to the hexasaccharide, preferably obtainable by a method according to any one of claims 8 or 9.

15. Higher oligosaccharides with a degree of polymerization of 9 - 13, consisting of a Glc(1→ ) Glc(1→ ) Glc(1→ ) βMan(1→4)βGlc(1→4) [βGal (1→2)αGal(1→6)]Man with three or more β-Gal monosaccharide residues attached as branches to the octasaccharide, preferably obtainable by a method according to any one of claims 8 or 9.
